# EUROPEAN PATENT APPLICATION

(11) **EP 4 516 206 A1**
(43) Date of publication of application: **05.03.2025**
(21) Application number: 23796001.8
(22) Date of filing: 30.03.2023
(51) Int. Cl.: A61B 3/10, G06T 7/00, G06T 7/11

(54) **OPHTHALMIC DEVICE, OPHTHALMIC IMAGE PROCESSING METHOD, AND RECORDING MEDIUM**

(30) Priority: 28.04.2022 JP 2022074096
(71) Applicant: TOPCON CORPORATION, Tokyo 174-8580 (JP)
(72) Inventor: ONO, Yusuke, Tokyo 174-8580 (JP)
(74) Representative: Gagel, Roland
(86) International application number: PCT/JP2023/013123
(87) International publication number: WO 2023/210254

(57) **Abstract**

An ophthalmic device pertaining to an exemplary embodiment example of the present invention includes a moving image acquisition unit, an image processing unit, and an evaluation processing unit. The moving image acquisition unit acquires a moving image of an eyelid of a subject. The image processing unit identifies a meibomian gland region from at least one frame of the moving image of the eyelid of the subject acquired by the moving image acquisition unit. The evaluation processing unit generates evaluation information about predetermined diseases on the basis of the meibomian gland region identified by the image processing unit.

## Description

### [TECHNICAL FIELD]

The present disclosure generally relates to an ophthalmic apparatus, a method of processing an ophthalmic image, and a recording medium.

### [BACKGROUND OF THE INVENTION]

Dry eye is a chronic disease of the lacrimal fluid and corneal conjunctival epithelium. There are various causes of dry eye, and typical examples thereof include abnormalities in the lipid layer due to meibomian gland dysfunction, abnormalities in the aqueous layer due to Sjögren's syndrome, and abnormalities in the mucin layer due to Stevens-Johnson syndrome or ocular cicatricial pemphigoid.

In diagnosis of dry eye, examination of meibomian glands is conducted. Meibomian glands are sebaceous glands that are present on the eyelids, with approximately 50 pieces on the upper eyelid and approximately 25 pieces on the lower eyelid. Sebum (oily substance) supplied from meibomian glands forms a lipid layer on the surface of the lacrimal fluid, preventing excessive evaporation of the lacrimal fluid. Meibography is known as a method of examining meibomian glands (refer to, for example, Patent Documents 1 and 2).

### [PRIOR ART DOCUMENTS]

### [PATENT DOCUMENTS]

[PATENT DOCUMENT 1] Japanese Unexamined Patent Application Publication No. 2009-285447
[PATENT DOCUMENT 2] Japanese Unexamined Patent Application Publication No. 2012-217621

### [BRIEF SUMMARY OF THE INVENTION]

### [PROBLEM TO BE SOLVED BY THE INVENTION]

Existing or conventional meibography-based examinations include a step of photographing the back surface of the eyelid, a step of saving a still image obtained by the photography, and a step of analyzing and evaluating the still image. Having these steps make it difficult to provide an examination result quickly.

One object of the present disclosure is to speed up the provision of an examination result of meibography.

### [MEANS FOR SOLVING THE PROBLEM]

An ophthalmic apparatus according to an exemplary embodiment includes a moving image acquisition unit, an image processor, and an evaluation processor. The moving image acquisition unit is configured to acquire a moving image of a subject's eyelid. The image processor is configured to identify a meibomian gland area from at least one frame of the moving image of the subject's eyelid acquired by moving image acquisition unit. The evaluation processor is configured to generate evaluation information relating to a predetermined disease based on the meibomian gland area identified by the image processor.

### [EFFECT OF THE INVENTION]

The exemplary embodiment according to the present disclosure is capable of speeding up the provision of an examination result of meibography.

### [BRIEF DESCRIPTION OF THE SEVERAL VIEWS OF THE DRAWING]

FIG. 1 is a block diagram illustrating the configuration of an ophthalmic apparatus according to an aspect example of an embodiment.
FIG. 2 is a flowchart illustrating processing performed by the ophthalmic apparatus according to the aspect example of the embodiment.
FIG. 3 is a flowchart illustrating processing performed by the ophthalmic apparatus according to the aspect example of the embodiment.
FIG. 4 is a block diagram illustrating the configuration of an ophthalmic apparatus according to the first embodiment example.
FIG. 5 is a block diagram for describing the configuration of the ophthalmic apparatus according to the first embodiment example.
FIG. 6 is a flowchart illustrating processing performed by the ophthalmic apparatus according to the first embodiment example.
FIG. 7 is a block diagram illustrating the configuration of an ophthalmic apparatus according to the second embodiment example.
FIG. 8 is a flowchart illustrating processing performed by the ophthalmic apparatus according to the second embodiment example.
FIG. 9 is a block diagram illustrating the configuration of an ophthalmic apparatus according to the third embodiment example.
FIG. 10 is a flowchart illustrating processing performed by the ophthalmic apparatus according to the third embodiment example.
FIG. 11 is a block diagram illustrating the configuration of an ophthalmic apparatus according to the fourth embodiment example.
FIG. 12 is a flowchart illustrating processing performed by the ophthalmic apparatus according to the fourth embodiment example.
FIG. 13 is a block diagram illustrating the configuration of an ophthalmic apparatus according to the fifth embodiment example.
FIG. 14 is a flowchart illustrating processing performed by the ophthalmic apparatus according to the fifth embodiment example.
FIG. 15 is a block diagram illustrating the configuration of an ophthalmic apparatus according to the sixth embodiment example.
FIG. 16 is a flowchart illustrating processing performed by the ophthalmic apparatus according to the sixth embodiment example.

### [DETAILED DESCRIPTION OF THE INVENTION]

Some embodiment examples according to the present disclosure will be described in detail with reference to the drawings.

Any known techniques or technologies can be combined with the embodiment examples according to the present disclosure. For example, any known techniques or technologies according to any technical fields related to the present disclosure, such as any matters or items described in the documents cited in the present disclosure, may be combined with the embodiment examples according to the present disclosure.

In addition, any matters or items disclosed by the applicant of the present application regarding any techniques or technologies related to the present disclosure (e.g., matters or items disclosed in patent applications, academic papers, etc.) may be combined with the embodiment examples according to the present disclosure.

Furthermore, any two or more of the various aspect examples of the embodiments according to the present disclosure may be combined.

It should be noted that a combination of at least two technical matters or items can refer to a combination of the entire technical matters or items or a combination of parts thereof.

One or more of the functions of the elements described in the embodiments according to the present disclosure can be implemented by using a circuit configuration (circuitry) or a processing circuit configuration (processing circuitry).

The circuitry or the processing circuitry includes any of the followings, all of which are configured and/or programmed to execute one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuit configuration or circuitry, and any combination of these.

A processor is considered to be processing circuitry or circuitry that includes a transistor and/or another circuitry.

In the present disclosure, circuitry, a unit, a means, or any terms similar to these is hardware configured to execute one or more functions disclosed herein, or hardware that is programmed to execute one or more functions disclosed herein. The hardware may be any hardware described in the embodiments according to the present disclosure, or alternatively, known hardware that is programmed and/or configured to execute one or more functions described herein. In the case where the hardware is a processor, which may be considered as a certain type of circuitry, then circuitry, a unit, a means, or any terms similar to these is a combination of hardware and software. In this case, the software is used to configure the hardware and/or the processor.

One or more of the functions of the elements described in the embodiments according to the present disclosure may be configured by means of artificial intelligence techniques or technologies such as machine learning.

The present disclosure describes some embodiments of an ophthalmic apparatus, some embodiments of a method of processing an ophthalmic image, some embodiments of a program, and some embodiments of a recording medium. However, embodiments are not limited to these categories.

### < Ophthalmic apparatus >

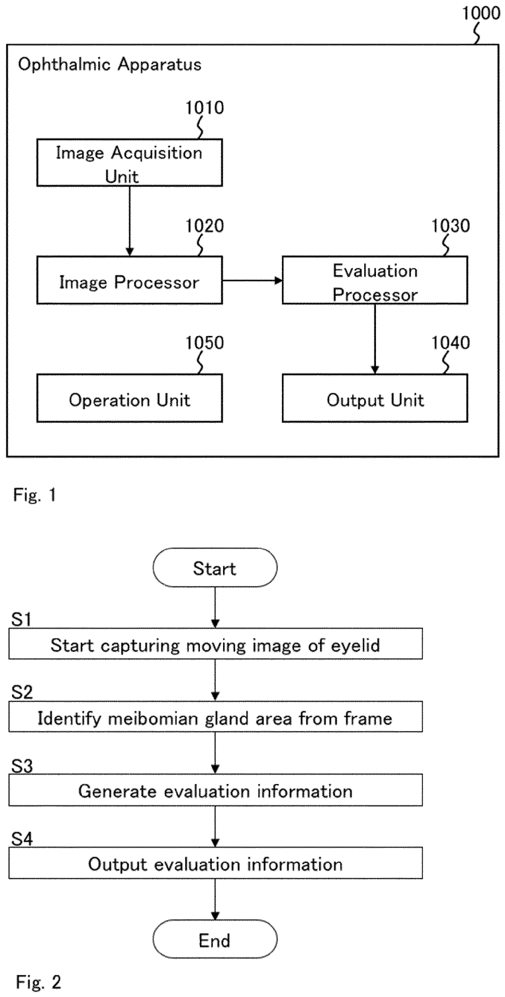

Some aspect examples of an embodiment of an ophthalmic apparatus will be described. The configuration of an ophthalmic apparatus according to one aspect example is shown in FIG. 1. The ophthalmic apparatus 1000 of the present aspect may be used at least for meibography.

The ophthalmic apparatus 1000 includes the image acquisition unit 1010, the image processor 1020, and the evaluation processor 1030. The ophthalmic apparatus 1000 may further include the output unit 1040 and the operation unit 1050 as optional elements.

Although not shown in the drawings, the ophthalmic apparatus 1000 may include one or more processors such as a control processor, an arithmetic processor (calculation processor), and a processing processor, and may include one or more storage devices such as a RAM, a ROM, a hard disk drive, or a solid state drive. Furthermore, the ophthalmic apparatus 1000 may include any elements other than those exemplified above.

### < Image acquisition unit 1010 >

The image acquisition unit 1010 is configured to acquire an image of the subject. The image acquired by the image acquisition unit 1010 of the present aspect is an image acquired in ophthalmology (ophthalmic image), and in particular, an image used for examination of meibomian glands. More specifically, the ophthalmic image of the present aspect is one or more frames of a moving image that depicts an image of a meibomian gland, and in particular, one or more frames obtained by applying moving image photography to the back surface of an eyelid (that is, the surface in contact with an eyeball).

The image acquisition unit 1010 may include any one or both of an imaging apparatus and a reception apparatus.

The imaging apparatus has a function of photographing a subject and generating an image thereof. The imaging apparatus may be an ophthalmic imaging apparatus. The imaging apparatus according to one aspect example includes an illumination system configured to be capable of outputting infrared light and a photographing system including a digital camera configured to be capable of performing moving image photography. The imaging apparatus according to one aspect example may be any existing or conventional ophthalmic imaging apparatus that can be used for meibography. In the case where the image acquisition unit 1010 includes such an imaging apparatus, it is possible to perform processing by the image processor 1020 and processing by the evaluation processor 1030 in almost real time for moving image photography performed by the imaging apparatus.

The reception apparatus has a function of accepting an image of a subject from an external apparatus or external device. The reception apparatus according to one aspect example includes a receiving device configured to receive an image from an imaging apparatus. In some examples, in parallel with moving image photography performed by the imaging apparatus, the reception apparatus accepts at least one of a plurality of frames collected in time series by this moving image photography. In the present aspect example, the latency between acquisition of a frame by the imaging apparatus and reception of this frame by the reception apparatus is very low, and therefore, it becomes possible to perform the processing by the image processor 1020 and the processing by the evaluation processor 1030 in almost real time with respect to the moving image photography performed by the imaging apparatus.

The reception apparatus according to another aspect example accepts an image generated by an imaging apparatus via another device (relay device). The relay device may include any kinds of devices, for example, may include any of a communication device, a storage device, a recording medium, a medical device, and a computer. In the present aspect example as well, by configuring the latency between acquisition of a frame by the imaging apparatus and reception of that frame by the reception apparatus to be very low, it becomes possible to perform the processing by the image processor 1020 and the processing by the evaluation processor 1030 in almost real time for the moving image photography performed by the imaging apparatus.

As described above, some aspect examples are configured in such a manner that the latency between photographing and processing is very low; however, embodiments according to the present disclosure are not limited to such configurations. For example, in some aspect examples, a configuration can be adopted in which improvement of processing quality (such as processing accuracy and precision) by the use of machine learning is prioritized over the real-time processing capability for photography (moving image photography or still image photography) of a subject. In one aspect example of such a configuration, images acquired by an imaging apparatus may be stored in a predetermined device (e.g., a medical information management system, a medical information archiving system, etc.), and a desired image may be read out from that device and processed by the ophthalmic apparatus 1000.

### < Image processor 1020 >

The image processor 1020 is configured to identify a meibomian gland area from an image acquired by the image acquisition unit 1010. The function of the image processor 1020 is implemented by cooperation between software such as an image processing program and hardware such as a processor.

In one aspect example, the image acquisition unit 1010 is configured to acquire a moving image of a subject's eyelid (e.g., the back surface of the eyelid), and the image processor 1020 is configured to identify a meibomian gland area from at least one frame of the moving image acquired.

Some examples of the processing performed by the image processor 1020 in order to identify a meibomian gland area in an image of a subject's eyelid, will be described later. This processing may, for example, utilize artificial intelligence techniques or technologies at least in part, or may not utilize artificial intelligence techniques or technologies. Some aspect examples of embodiments that utilize artificial intelligence techniques or technologies will be described later.

As for an embodiment that does not utilize artificial intelligence techniques or technologies, the image processor 1020 according to one aspect example may be configured to perform the processing disclosed in Japanese Unexamined Patent Application Publication No. 2012-217621 (Patent Document 2).

The image processor 1020 of the present aspect is configured to perform processing of designating an analysis target area for an eyelid image and processing of extracting a meibomian gland area from the eyelid image based on the brightness value of each pixel in the analysis target area. The analysis target area is a predetermined area of the eyelid that indicates an area from which the meibomian gland area is to be extracted. The analysis target area may be a part or the entirety of the image of the eyelid depicted in the eyelid image.

The designation of the analysis target area is performed based on the pixel values of the eyelid image. For example, the image processor 1020 of the present aspect may be configured to identify the outer edge of the image area corresponding to the back surface of the eyelid based on the brightness value of each pixel in the eyelid image, and designate the area surrounded by the identified outer edge as an analysis target area. Alternatively, the image processor 1020 of the present aspect is configured to designate an analysis target area to be an area that is likely to contain a meibomian gland, by identifying a part having relatively high brightness from the entirety of an image of an eyelid depicted in an eyelid image.

Furthermore, the image processor 1020 of the present aspect may be configured to extract a meibomian gland area based on the brightness of each pixel in the analysis target area designated. The processing of extracting a meibomian gland area may include any kinds of image processing techniques, and may include, for example, segmentation (area division), filtering, region growing, or the like.

Contrast enhancement, sharpening, or the like may be performed as preprocessing for the meibomian gland area extraction.

The image processor 1020 of the present aspect may be configured to identify a meibomian gland area from the eyelid image by the processing as described above, without the use of artificial intelligence techniques or technologies.

In one aspect example, the image acquisition unit 1010 is configured to acquire a moving image of a subject's eyelid (e.g., the back surface of the eyelid), and the image processor 1020 is configured to identify a meibomian gland area from each of the plurality of frames of the acquired moving image by sequentially processing the plurality of frames of the moving image.

By identifying meibomian gland areas from the plurality of frames (the plurality of eyelid images), it becomes possible to selectively adopt an eyelid image in which a meibomian gland is well depicted. This improves the quality (e.g., accuracy, precision, etc.) of the meibomian gland examination. In addition, the possibility of having to retake images due to blinking or other obstructions can be reduced.

### < Evaluation processor 1030 >

The evaluation processor 1030 is configured to generate evaluation information relating to dry eye, based on the meibomian gland area identified from the eyelid image by the image processor 1020. The evaluation information is provided for the diagnosis of dry eye and, in a broader sense, for the diagnosis of any kinds of diseases based on the condition of the meibomian glands. The function of the evaluation processor 1030 is implemented by cooperation between software such as an evaluation processing program and hardware such as a processor.

The evaluation processor 1030 is configured to generate one or more predetermined types of evaluation information. In the case of generating two or more types of evaluation information, the evaluation processor 1030 may be configured to always generate the two or more types of evaluation information, may be configured to selectively generate the two or more types of evaluation information, or may be configured to always generate one or more type of evaluation information and optionally and/or selectively generate one or more other types of evaluation information. Some examples of the types of evaluation information are described below.

In some aspect examples, the evaluation processor 1030 is configured to generate evaluation information obtained by quantifying the area of meibomian gland loss (dropout area). Examples of such evaluation information include known meiboscores and known meiboscales. These examples of evaluation information are used in the diagnosis of dry eye.

The evaluation information of these examples is generated by acquiring an image of the back surface of an eyelid by means of meibography, calculating the proportion (percentage) of the area of meibomian gland loss from the image acquired, and assigning a grade corresponding to the proportion calculated. Although the number of grades or the range of the percentage of the area of loss corresponding to each grade differ between evaluation methods, processing performed by the evaluation processor 1030 is fundamentally the same.

For these evaluation methods, refer to, for example, the following literature: Reiko Arita, "Meibography: A Japanese Perspective", Investigative Ophthalmology & Visual Science, November 2018, Volume 59, Issue 14.

It should be noted that existing or conventional methods for generating the evaluation information in the above examples do not conduct processing of a frame of a moving image in the same way as the present embodiment does. Instead, existing or conventional methods include a step of photographing the back surface of an eyelid, a step of saving a still image obtained by the photography, and a step of analyzing and evaluating the still image. Therefore, existing or conventional methods have the problem of not being able to provide an examination result quickly.

Abnormalities of the meibomian glands include not only loss but also shortening, tortuosity (bending), dilation. Knowledge regarding abnormalities in meibomian glands includes, for example, the following pieces of information: the area of meibomian gland loss increases with age; the meibomian glands of patients with obstructive meibomian gland dysfunction are significantly lost, shortened, bent, or dilated compared to normal eyes; the meibomian glands of eyes wearing contact lenses are significantly shortened compared to normal eyes; the meibomian glands of patients with perennial allergic conjunctivitis are significantly more bent compared to normal eyes; the meibomian glands of patients to whom anti-glaucoma eye drops have been administered for a long time are significantly more lost; the meibomian glands of patients after ocular radiation therapy or trabeculectomy (glaucoma surgery) are prone to atrophy or loss; the meibomian glands of patients with granular corneal dystrophy (type II) are prone to loss; the meibomian glands of patients with chalazion are visualized as low-reflective by meibography; and the meibomian glands of patients with sebaceous carcinoma are visualized as high-reflective by meibography.

Based on the knowledge described above, evaluation information and evaluation methods can be designed. Some examples of such evaluation information and evaluation methods are disclosed in Japanese Unexamined Patent Application Publication No. 2012-217621 (Patent Document 2), and it will be understood by those skilled in the art that evaluation information and evaluation methods other than the examples disclosed may also be designed by using the same or similar methods.

### < Output Unit 1040 >

The output unit 1040 is configured to perform output of information. For example, the output unit 1040 may be configured to perform output of an image acquired by the image acquisition unit 1010, output of information generated by the image processor 1020, output of information generated by the evaluation processor 1030, and so forth.

The output unit 1040 of some examples operates under control of a processor (control unit) not shown in the drawings. This processor is configured, for example, to perform control of the output unit 1040 by means of a control program not shown in the drawings.

The output unit 1040 includes, for example, at least one of a communication device, a display device, an audio output device, a printing device, and a media drive.

The communication device may include a modem, a router, a communication circuit, and so forth. The communication device transmits information to be output toward other devices or other systems. The communication device may be implemented by using the communication function of a specified computer. This computer may be, for example, a smartphone, a tablet computer, a laptop computer, a desktop computer, an Internet of Things device (IoT device), or the like.

The display device may include a liquid crystal display, a display circuit, and so forth. The display device is configured to display visual information based on information to be output.

The audio output device may include an amplifier, a speaker, an audio output circuit, and so forth. The audio output device is configured to emit auditory information based on information to be output.

The printing device may include a printer, a printing circuit, and so forth. The printing device is configured to form visual information on paper based on information to be output.

The media drive may include a data writer, a data writing circuit, or the like that are configured to write data into a computer-readable non-transitory recording medium. The media drive is configured to record information to be output onto a recording medium. This recording medium may be in any form, and examples thereof include a magnetic disk, an optical disk, a magneto-optical disk, and a semiconductor memory.

### < Operation unit 1050 >

The operation unit 1050 has a configuration used for performing operation or manipulation of the ophthalmic apparatus 1000 and/or performing input of information. The operation unit 1050 includes an operation device, an input device, or the like, such as a keyboard, a pointing device, or a console.

### < Operation >

Operations of the ophthalmic apparatus 1000 will now be described. Any two or more of the operation examples described below can be combined at least in part.

One example of the operation of the ophthalmic apparatus 1000 is shown in FIG. 2. In the first step of the present operation example, the ophthalmic apparatus 1000 starts moving image photography of the subject's eyelid (the back surface thereof) by the use of the image acquisition unit 1010 in response to receiving an instruction from the user (S1). This moving image photography is, for example, infrared moving image photography in which infrared light is used as illumination light. The ophthalmic apparatus 1000 displays, for example, a moving image (referred to as an observation image, or the like) generated by this moving image photography, on the display device of the output unit 1040 in real time.

Next, the ophthalmic apparatus 1000 identifies, by using the image processor 1020, a meibomian gland area from each of one or more frames of the moving image generated by the moving image photography started in the step S1 (S2). Several examples of the processing performed in the step S2 will be described below.

The first example of the step S2 will now be described. The user issues an instruction by means of the operation unit 1050 at a desired timing while referring to the observation image displayed on the display device. The ophthalmic apparatus 1000 selects at least one frame corresponding to the time point of the issue of the instruction, from among a group of frames sequentially acquired as the observation image. The image processor 1020 performs processing of each frame selected, thereby identifying a meibomian gland area from each of the at least one frame selected.

According to the present example, the user can refer to the displayed observation image and input the above instruction at a timing when the back surface of the subject's eyelid is appropriately depicted in the displayed observation image. This makes it possible to increase the possibility of obtaining a meibomian gland area suitable for evaluation.

The second example of the step S2 will now be described. The ophthalmic apparatus 1000 (the image processor 1020, or a processor not shown in the drawings) of the present example analyzes frames sequentially generated by the moving image photography started in the step S1 and detects that a frame satisfying a predetermined image quality condition has been obtained. The image processor 1020 performs processing of at least one frame including the frame satisfying the predetermined image quality condition, to identify a meibomian gland area from each of the at least one frame.

According to the present example, a meibomian gland area can be automatically identified from the frame satisfying the predetermined image quality condition, which increases the possibility of obtaining a meibomian gland area suitable for evaluation.

It should be noted that the image quality condition is determined in advance so that the identification of a meibomian gland area can be performed in a suitable manner, and that the image quality condition may be determined based on a freely selected or determined image parameter(s) (e.g., brightness, contrast, signal-to-noise ratio, sharpness, etc.).

The third example of the step S2 will be now described. The ophthalmic apparatus 1000 of the present example performs real-time identification and real-time display of a meibomian gland area, by performing real-time processing of the observation image generated by the moving image photography started in the step S1 by means of the image processor 1020. The user issues an instruction by the use of the operation unit 1050 at a desired timing while referring to a real-time image of the meibomian gland area displayed on the display device. The ophthalmic apparatus 1000 selects at least one meibomian gland area corresponding to the time point of the issue of the instruction, and sends the selected meibomian gland area(s) to the evaluation processor 1030.

According to the present example, the user can input the instruction at a timing when the meibomian glands are appropriately depicted, by referring to the displayed real-time image of the meibomian gland area. This increases the likelihood that a meibomian gland area suitable for evaluation will be provided to the evaluation processor 1030.

The fourth example of the step S2 will be now described. The ophthalmic apparatus 1000 (the image processor 1020, or a processor not shown in the drawings) of the present example performs real-time processing of the observation image being generated by the moving image photography started in the step S1 to conduct real-time identification of a meibomian gland area. In addition to this, the ophthalmic apparatus 1000 analyzes meibomian gland areas sequentially identified, thereby detecting that a meibomian gland area satisfying a predetermined image quality condition has been obtained. Furthermore, the ophthalmic apparatus 1000 selects at least one meibomian gland area including the meibomian gland area satisfying the predetermined image quality condition detected in this way and sends the selected meibomian gland area(s) to the evaluation processor 1030.

According to the present example, a meibomian gland area satisfying the predetermined image quality condition can be automatically identified, thereby increasing the possibility of obtaining a meibomian gland area suitable for evaluation.

Next, by using the evaluation processor 1030, the ophthalmic apparatus 1000 generates evaluation information relating to a predetermined disease based on the meibomian gland area identified in the step S2 (S3).

Finally, the ophthalmic apparatus 1000 outputs the evaluation information generated in the step S3 by using the output unit 1040 (S4). In the present step, any one or more of the following pieces of information may be output together with the evaluation information generated in the step S3: one or more frames acquired by the moving image photography started in the step S1; information on the meibomian gland area identified in the step S2; and other predetermined information. With the above, the operation example in FIG. 2 comes to an end (End).

According to the present embodiment which is configured to be capable of performing the operation example of FIG. 2, a meibomian gland area can be identified from a frame of a moving image of the subject's eyelid, and evaluation information can be generated. In contrast, existing or conventional examinations with meibography require the following steps: a step of photographing the back surface of the eyelid; a step of storing a still image obtained by the photography; and a step of analyzing and evaluating the still image. Therefore, according to the present embodiment which is configured to be capable of performing the operation example of FIG. 2, it is possible for the present embodiment to provide an examination result relating to a meibomian gland more quickly than with the existing or conventional techniques or technologies.

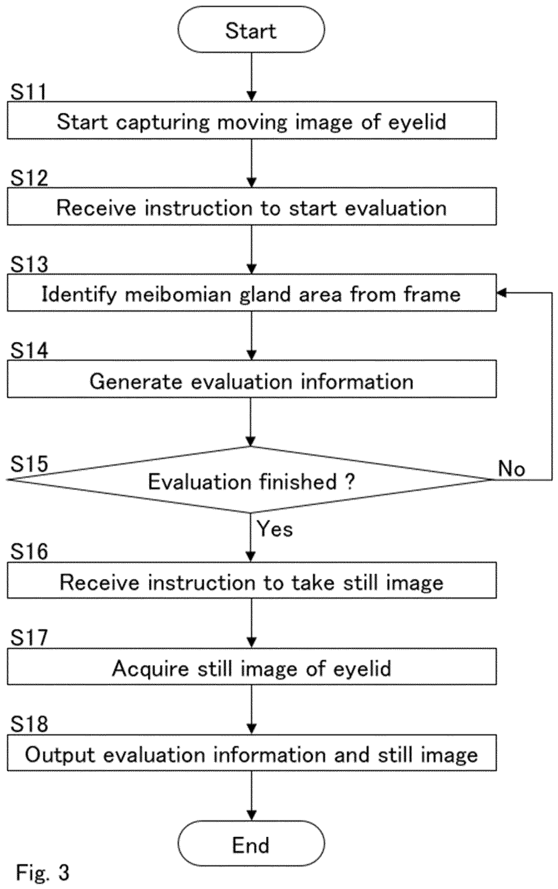

Another example of the operation of the ophthalmic apparatus 1000 is shown in FIG. 3. In the first step of the present operation example, the ophthalmic apparatus 1000 starts moving image photography of the subject's eyelid (the back surface thereof) in response to the reception of an instruction from the user (S11).

The moving image photography of the present example may be infrared moving image photography using infrared light for illumination, for example. The ophthalmic apparatus 1000 displays, for example, a moving image (referred to as an observation image, or the like) generated by this moving image photography, on the display device of the output unit 1040 in real time.

In some aspect examples, the user inputs an instruction to start evaluation of the meibomian glands (an evaluation start instruction) at a desired timing by means of the operation unit 1050 while referring to the observation image displayed on the display device.

In some other aspect examples, the ophthalmic apparatus 1000 (the image processor 1020, or a processor not shown in the drawings) performs real-time processing of the observation image being generated by the moving image photography started in the step S1, thereby making a determination in real time whether a frame of the observation image satisfies a predetermined image quality condition. This image quality condition may be the same as or similar to the image quality condition in the operation example of FIG. 2, or may be different from those. The ophthalmic apparatus 1000 (the image processor 1020, or a processor not shown in the drawings) issues a predetermined signal (an evaluation start instruction) in response to the acquisition of a frame that satisfies the image quality condition.

The evaluation start instruction is not limited to the two examples described above. When the ophthalmic apparatus 1000 receives either of the evaluation start instructions (S12), it then starts the processing of the next step S13.

Upon receiving the evaluation start instruction, the ophthalmic apparatus 1000 identifies, by using the image processor 1020, a meibomian gland area from one frame generated after the issue of the evaluation start instruction by the moving image photography started in the step S11 (S13). This processing may be the same as or similar to the processing performed in the step S2 of the operation example shown in FIG. 2, or may be different from that.

Next, the ophthalmic apparatus 1000 generates evaluation information relating to a predetermined disease based on the meibomian gland area identified from the frame in the step S13 by the evaluation processor 1030 (S14).

The processing of the step S13 and the processing of the step S14 are repeatedly performed until it is determined in the step S15 that "evaluation finished" (S15: No). More specifically, when it is determined as "No" in the step S15, the ophthalmic apparatus 1000 identifies a meibomian gland area from one newly generated frame (S13: the image processor 1020), and generates evaluation information based on the new meibomian gland area identified from the new frame (S14: the evaluation processor 1030).

Through the repetitive execution of the meibomian gland area identification and the evaluation information generation in this way, a plurality of pieces of evaluation information is sequentially generated in real time in parallel with the acquisition of the observation image (in parallel with the moving image photography). The plurality of pieces of evaluation information generated in this manner is stored in a storage device (not shown in the drawings).

In the step S15 of some aspect examples, the user inputs a predetermined instruction (an evaluation finish instruction) by using the operation unit 1050. Having received the evaluation finish instruction, the ophthalmic apparatus 1000 determines that the evaluation is ended and issues "evaluation finished" (S15: Yes).

In the step S15 of some other aspect examples, the ophthalmic apparatus 1000 may make a determination on whether the evaluation is finished or not based on at least one of the following pieces of data or information: one or more frames acquired by the moving image photography started in the step S11; one or more meibomian gland areas identified in the step S13; and one or more pieces of evaluation information generated in the step S14. Some examples of the determination of the present aspect examples are described below.

The ophthalmic apparatus 1000 of some examples may be configured to determine "evaluation finished" when a frame satisfying a predetermined image quality condition is acquired. The ophthalmic apparatus 1000 of some examples may be configured to determine "evaluation finished" when a predetermined number of consecutive frames each satisfying a predetermined image quality condition are acquired. The ophthalmic apparatus 1000 of some examples may be configured to determine "evaluation finished" when a meibomian gland area satisfying a predetermined image quality condition is acquired. The ophthalmic apparatus 1000 of some examples may be configured to determine "evaluation finished" when meibomian gland areas each satisfying a predetermined image quality condition are acquired from a predetermined number of consecutive frames. The ophthalmic apparatus 1000 of some examples may be configured to determine "evaluation finished" when a plurality of pieces of evaluation information with a stable condition is acquired from a predetermined number of consecutive frames. As a specific example thereof, the ophthalmic apparatus 1000 may be configured to determine "evaluation finished" when the same grade of meiboscore is obtained from a predetermined number of consecutive frames. It is possible to make determination of evaluation completion by combining, at least in part, any two or more of the plurality of examples described above. In addition, it is possible to make determination of evaluation completion by combining, at least in part, any one or more of the plurality of examples described above with processing other than these examples. It is also possible to make determination of evaluation completion by processing different from any of the plurality of examples described above.

After the determination of evaluation completion is made in the step S15 (S15: Yes), the ophthalmic apparatus 1000 receives an instruction to perform photography (still image photography) to obtain a still image of the subject's eyelid (the back surface thereof) (S16).

In some aspect examples, the user inputs an instruction to perform the still image photography by using the operation unit 1050. In some other aspect examples, the ophthalmic apparatus 1000 determines whether or not the observation image satisfies a predetermined image quality condition, and issues an instruction to perform the still image photography when the observation image is determined to satisfy the predetermined image quality condition. It should be noted that the instruction to perform the still image photography is not limited to the above examples.

The ophthalmic apparatus 1000, which has received the instruction to perform the still image photography, acquires a still image of the eyelid by using the image acquisition unit 1010 (S17). As mentioned above, the image acquisition unit 1010 may include any one or both of an imaging apparatus and a reception apparatus.

In the case where the image acquisition unit 1010 includes the imaging apparatus, the ophthalmic apparatus 1000 can generate a still image of the subject's eyelid by performing control of the imaging apparatus in response to the reception of the instruction to perform the still image photography.

In the case where the image acquisition unit 1010 includes the reception apparatus, the ophthalmic apparatus 1000 can transmit a still image photography instruction to an external imaging apparatus via the reception apparatus (e.g., a communication device) in response to the reception of the instruction to perform the still image photography. Then, the ophthalmic apparatus 1000 can receive, via the reception apparatus, a still image generated by the external imaging apparatus in response to the still image photography instruction.

The ophthalmic apparatus 1000 outputs, by using the output unit 1040, the evaluation information generated in the step S14 and the still image acquired in the step S17 (S18).

In the present operation example, a plurality of pieces of evaluation information are acquired by the repetition of the processing of the step S13 and the processing of the step S14. The evaluation information output in the step S18 may be all of the plurality of pieces of evaluation information acquired (the first case), may be one or more of the plurality of pieces of evaluation information (the second case), or may be evaluation information generated from one or more or all of the plurality of pieces of evaluation information (the third case).

In the first case, the ophthalmic apparatus 1000 may output a list of the plurality of pieces of acquired evaluation information by using the output unit 1040, for example. In some aspect examples, the ophthalmic apparatus 1000 may output a list of the plurality of meiboscores acquired.

In the second case, the ophthalmic apparatus 1000 may, for example, select one or more pieces of evaluation information according to a predetermined criterion from among the plurality of pieces of evaluation information acquired, and then output the one or more pieces of selected evaluation information by the output unit 1040. As a specific example, the ophthalmic apparatus 1000 may be configured to select the evaluation information that is closest to the average of the plurality of pieces of evaluation information, select the most frequent information from among the plurality of pieces of evaluation information, or select the information that is located in the middle when the plurality of pieces of evaluation information is arranged according to a predetermined criterion. In some aspect examples, the ophthalmic apparatus 1000 may be configured to select one or more meiboscores from among the plurality of meiboscores acquired, and then output the one or more meiboscores selected.

In the third case, the ophthalmic apparatus 1000 may generate evaluation information by applying statistical calculation to the plurality of pieces of evaluation information acquired, for example. The evaluation information obtained by the statistical calculation may be statistical information of any kind, such as an average value, a mode value, a median value, a maximum value, a minimum value, or other statistics. In some aspect examples, the ophthalmic apparatus 1000 may apply statistical calculation to the plurality of meiboscores acquired, and output the statistics calculated by the statistical calculation.

With the above, the operation example in FIG. 3 comes to an end (End).

According to the present embodiment which is configured to be capable of performing the operation example of FIG. 3, it is possible to identify a meibomian gland area in real time from a frame of the moving image of the subject's eyelid, and then generate evaluation information. In contrast, existing or conventional examinations conducted using meibography require the following steps: a step of photographing the back surface of the eyelid; a step of storing the still image obtained; and a step of analyzing and evaluating the still image. Therefore, the present embodiment configured to be capable of performing the operation example of FIG. 3 can provide an examination result relating to a meibomian gland more quickly than with the existing or conventional techniques or technologies.

Furthermore, according to the present embodiment which is configured to be capable of performing the operation example of FIG. 3, it is possible for the present embodiment to provide a still image of the eyelid in addition to the evaluation information. In the field of ophthalmic apparatuses (ophthalmic imaging apparatuses), it is typically the case that the quality of a still image is higher than the quality of each frame of a moving image. Also, while each frame of a moving image obtained by meibography is an infrared image and therefore a monochrome image, a still image may be a color image. Further, a high-quality monochrome image and a color image may be acquired as still images. Thus, the present embodiment configured to be capable of performing the operation example of FIG. 3 has an advantageous effect of not only being able to provide evaluation information quickly but also being able to provide a still image suitable for diagnosis and observation.

### < Embodiment examples of image processing >

As mentioned above, the processing performed by the image processor 1020 to identify a meibomian gland area in an image of the subject's eyelid, may utilize artificial intelligence technology at least in part. Some embodiment examples will be described below.

Any matters or items relating to the ophthalmic apparatus 1000 described above can be combined with the embodiment examples at least in part. It is also possible to combine different embodiment examples at least in part.

### < First embodiment example >

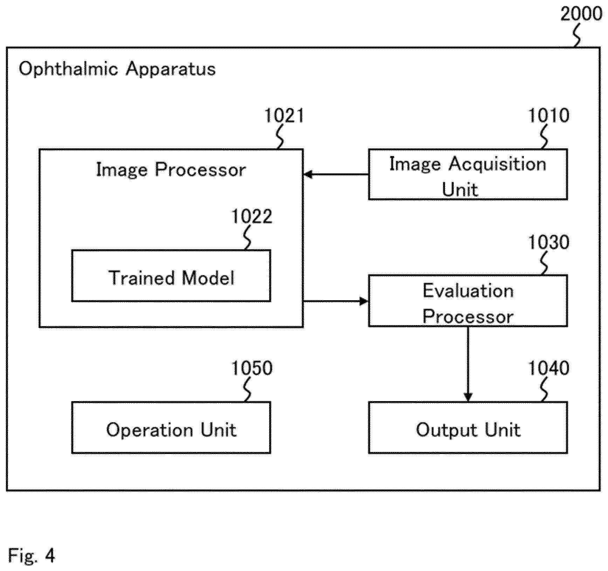

The first embodiment example will now be described. The ophthalmic apparatus 2000 shown in FIG. 4 includes the image acquisition unit 1010 and the evaluation processor 1030, which are the same as or similarly configured to those of the ophthalmic apparatus 1000 in FIG. 1. The image processor 1021 in FIG. 4 includes the trained model 1022. It should be noted that a trained model of some embodiment examples may be included in the image processor 1021 while a trained model of some other embodiment examples may be stored in a storage device to which the image processor can be accessible directly or indirectly. For example, a trained model can be placed in a computer (such as a server) connected to an ophthalmic apparatus of some embodiment examples via a communication line.

The ophthalmic apparatus 2000 may include, as optional elements, the output unit 1040 and the operation unit 1050, which are the same as or similarly configured to those of the ophthalmic apparatus 1000 in FIG. 1. Although not shown in the drawings, the ophthalmic apparatus 2000 may include a processor such as a control processor, an arithmetic processor (calculation processor), and a processing processor, and may also include a storage device such as a RAM, a ROM, a hard disk drive, of a solid state drive. Furthermore, the ophthalmic apparatus 2000 may include elements other than those exemplified above.

The image processor 1021 is configured to perform at least a part of the processing of identifying a meibomian gland area from a frame of a moving image of the subject's eyelid acquired by the image acquisition unit 1010, by using the trained model 1022.

The trained model 1022 is constructed by machine learning conducted using training data (learning data) that includes eyelid images. The trained model 1022 may be updated in a periodic or non-periodic basis. Eyelid images are obtained, for example, by photographing an eyelid (the back surface thereof) of a living body (human). Training data of some examples includes a large number of eyelid images collected from a large number of living bodies. Also, training data of some examples may include processed images generated by applying computer processing to eyelid images collected from living bodies (data augmentation).

The training data may include information (labels) attached to eyelid images by annotation performed by medical specialists. Also, the training data may include information (labels) attached to eyelid images by annotation executed by a computer. The labels are information corresponding to both the type of information input to the trained model 1022 and the type of information output from the trained model 1022.

The trained model 1022 constructed using the training data described above performs inference based on input data, and outputs resulting data. The combination of input data and output data may be, for example, any of the following options: a combination of an eyelid image as input data and an analysis target area as output data; a combination of at least part of an eyelid image as input data (e.g., the entirety of an eyelid image, an analysis target area, or the like) and a plurality of target areas as output data (e.g., areas of a biological tissue(s) identified by segmentation (image areas to be identified as a meibomian gland area, a corneal area, an eyelash area, or the like)); a combination of part of an eyelid image (e.g., an analysis target area) as input data and a meibomian gland area as output data; and a combination of the entirety of an eyelid image as input data and a meibomian gland area as output data. In any of these examples, the input data is an eyelid image (a frame of a moving image of the subject's eyelid) or an image obtained based on an eyelid image.

The image processor 1021 of the present embodiment example sends, to the evaluation processor 1030, at least part of the output data generated by the trained model 1022 and/or data (processed data) generated by applying processing to at least part of the output data generated by the trained model 1022.

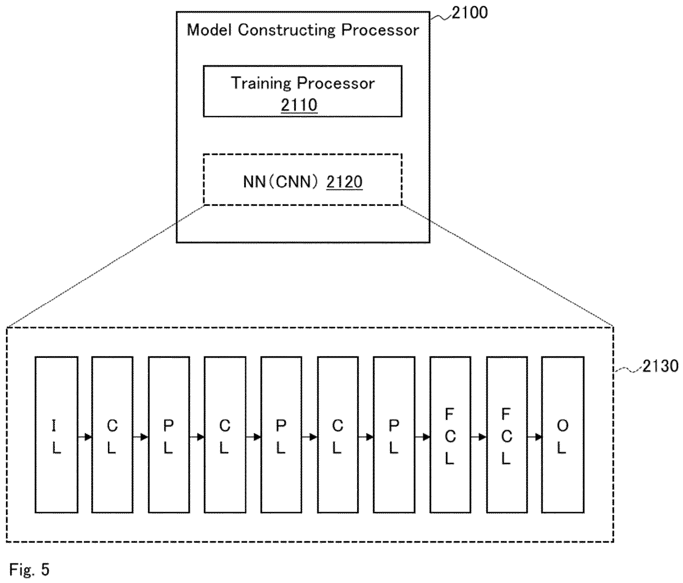

The trained model 1022 (at least a part thereof) is a mathematical model for processing images, and may be constructed by the model constructing processor 2100 shown as an example in FIG. 5. The model constructing processor 2100 of the present example includes the training processor 2110 and the neural network 2120.

The neural network 2120 includes, for example, a convolutional neural network (CNN). The reference character 2130 in FIG. 5 indicates an example of the structure of the convolutional neural network. It should be noted that the neural network 2120 may include a neural network of another type.

An image is input into the input layer of the convolution neural network 2130 of the present example. Behind the input layer, a plurality of pairs of a convolutional layer and a pooling layer is disposed. The convolution neural network 2130 of the present example includes three pieces of pairs of a convolution layer and a pooling layer; however, the number of the pairs of a convolution layer and a pooling layer may be freely selected or determined.

In the convolutional layer, a convolution operation is performed to detect or extract a feature (e.g., contour) from the input image. This convolution operation is a multiply-accumulate operation (a multiply-add operation, a product-sum operation) on the input image. This multiply-accumulate operation is performed with a filter function (a weight coefficient, a filter kernel) having the same dimension as the input image. In the convolutional layer, the convolution operation is applied to individual parts (individual sections, individual portions) of the input image. More specifically, the convolutional layer is configured to calculate a product by multiplying the value of each pixel in a partial image, to which the filter function has been applied, by the value (weight) of the filter function corresponding to this pixel, and then calculate the sum of the products over a plurality of pixels in this partial image. The sum of products obtained in this way is substituted for the corresponding pixel in an image to be output from the convolutional layer. By repetitively performing such multiply-accumulate operation in parallel with moving sites (parts) to which the filter function is applied (that is, in parallel with changing or switching partial images of the input image), a result of the convolution operation for the entire input image is obtained. The convolution operation performed in this way gives a large number of images in which various features have been extracted using a large number of weight coefficients. This means that a large number of filtered images, such as smoothed images and edge images, are obtained. The large number of images generated by the convolutional layer are referred to as feature maps (or activation maps).

The pooling layer executes data compression (e.g., data thinning) of the feature maps generated by the convolutional layer disposed at the immediately preceding position. More specifically, the pooling layer calculates statistical values in predetermined neighboring pixels of a predetermined pixel of interest in an input feature map at each predetermined pixel intervals, and outputs an image having a size smaller than the input feature map. The statistical values applied to the pooling operation may be maximum values (max pooling) or average values (average pooling), for example. The value of the pixel intervals applied to the pooling operation is referred to as a stride.

Typically, a convolutional neural network extracts many features from an input image by executing processing using a plurality of pairs of a convolutional layer and a pooling layer.

A fully connected layer is disposed behind the most downstream pair of a convolutional layer and a pooling layer. While two pieces of fully connected layers are included in the convolution neural network 2130 of the present example, the number of fully connected layers may be freely selected or determined. The fully connected layer executes predetermined processing (e.g., information generation such as image classification, image segmentation, or regression) based on the features compressed by the combination of convolution and pooling. An output layer is disposed behind the most downstream fully connected layer. The output layer gives an output result.

Some aspect examples may employ a convolutional neural network including no fully connected layer. For example, some aspect examples may employ a fully convolutional network (FCN). Also, some aspect examples may include a support vector machine, a recurrent neural network (RNN), or any other models. Further, machine learning applied to the neural network 2120 may include transfer learning. In other words, the neural network 2120 may include a neural network that has already been trained using other training data (such as training images) and whose parameters have been adjusted (tuned). Further, the model constructing processor 2100 (the training processor 2110) may be configured in such a manner that fine tuning can be applied to a trained neural network (the neural network 2120). The neural network 2120 may be constructed, for example, by means of a known open source neural network architecture.

The training processor 2110 applies machine learning with training data to the neural network 2120. In the case in which the neural network 2120 includes a convolutional neural network (e.g., the convolutional neural network 2130), parameters tuned by the training processor 2110 include, for example, filter coefficients of one or more convolutional layers therein and connection weights and offsets of one or more fully connected layers therein.

The training method or technique (machine learning method or technique) used to construct the neural network 2120 included in the trained model 1022 may be freely selected or determined, and may include any of supervised learning, unsupervised learning, and reinforcement learning, or may include a combination of any two or more of these.

In order to prevent the overconcentration of processes in a specific unit of the neural network 2120, the training processor 2110 may randomly select and invalidate one or more units of the neural network 2120 and execute learning using the remaining units. Such a function is referred to as dropout.

Some aspect examples are configured to conduct supervised learning using training data generated by annotation in which labels are assigned to input images. The annotation is performed by, for example, assigning, to each image included in the training data, a label determined based on that image.

In the annotation of the first example, assigned to an eyelid image is a label indicating an analysis target area which is a partial area of that eyelid image.

In the annotation of the second example, assigned to the entirety of an eyelid image are a plurality of labels respectively indicating a plurality of target areas, each of which is a partial area of that eyelid image. Here, the target areas are, for example, image areas that should be identified as meibomian gland areas, cornea areas, eyelash areas, or the like.

In the annotation of the third example, assigned to a partial area (e.g., an analysis target area) of the eyelid image are a plurality of labels respectively indicating a plurality of target areas, each of which is a further partial area of that partial area. Here, the target areas are, for example, image areas that should be identified as meibomian gland areas, cornea areas, eyelash areas, or the like.

In the annotation of the fourth example, assigned to a partial area (e.g., an analysis target area) of the eyelid image is a label indicating a meibomian gland area which is a further partial area of that partial area.

In the annotation of the fifth example, assigned to the entirety of an eyelid image is a label indicating a meibomian gland area which is a partial area of that eyelid image.

Operations of assigning labels as described above (labeling) may be performed, for example, by at least one of a doctor, a computer, and another mathematical model. The training processor 2110 can construct the trained neural network 2120 by applying supervised learning with training data thus created to the neural network 2120, and the trained model 1022 constructed can be created using the trained neural network 2120.

The method or technique used to the construction of the trained model 1022 is not limited to the examples shown above. For example, any methods and techniques such as the following options may be employed for creating the trained model 1022: support vector machine, Bayes classifier, boosting, k-means clustering, kernel density estimation, principal component analysis, independent component analysis, self-organizing map (or self-organizing feature map), random forest (or randomized trees, random decision forests), and generative adversarial network (GAN).

The mathematical model included in the trained model 1022 is not limited to a convolutional neural network. The mathematical model included in the trained model 1022 may include at least one of the following types of mathematical models: a convolutional neural network, a neural network of a type other than a convolutional neural network, and a mathematical model of a type other than a neural network.

As mentioned above, the image processor 1021 according to the present embodiment example performs, using the trained model 1022, at least part of the processing of identifying a meibomian gland area from a frame of a moving image of the subject's eyelid acquired by the image acquisition unit 1010.

The trained model 1022 generated by the use of any of the annotations of the first to fourth examples described above is used only in part of the meibomian gland identification processing performed by the image processor 1021, while the trained model 1022 generated using the annotation of the fifth example is used in the entirety of the meibomian gland identification processing performed by the image processor 1021.

More specifically, each trained model 1022 is used as follows: the trained model 1022 generated by means of the annotation of the first example can be employed for the processing of determining an analysis target area in the entirety of a frame; the trained model 1022 generated by means of the annotation of the second example is employed for the processing of determining a plurality of segments (a plurality of image areas) of the entirety of a frame; the trained model 1022 generated by means of the annotation of the third example is employed for the processing of determining a plurality of segments (a plurality of image areas) of a part of a frame (e.g., an analysis target area of a frame); the trained model 1022 generated by means of the annotation of the fourth example is employed for the processing of determining a meibomian gland area in a part of a frame (e.g., an analysis target area of a frame); and the trained model 1022 generated by means of the annotation of the fifth example is employed for the processing of determining a meibomian gland area in the entirety of a frame.

In this way, the image processor 1021 of the present embodiment example may be configured to perform only processing that uses a mathematical model that has been trained by machine learning, or it may be configured to perform both processing that uses a mathematical model that has been trained by machine learning and rule-based processing on the basis of a predefined algorithm.

It should be noted that in the invention disclosed in Japanese Unexamined Patent Application Publication No. 2012-217621 (Patent Document 2), the processing of identifying a meibomian gland area from a still image (photographed image) of the back surface of an eyelid is performed using only rule-based processing. In other words, the invention disclosed in Japanese Unexamined Patent Application Publication No. 2012-217621 (Patent Document 2) is configured to identify a meibomian gland area from a still image (photographed image) of the back surface of an eyelid without using a mathematical model that has been trained by machine learning.

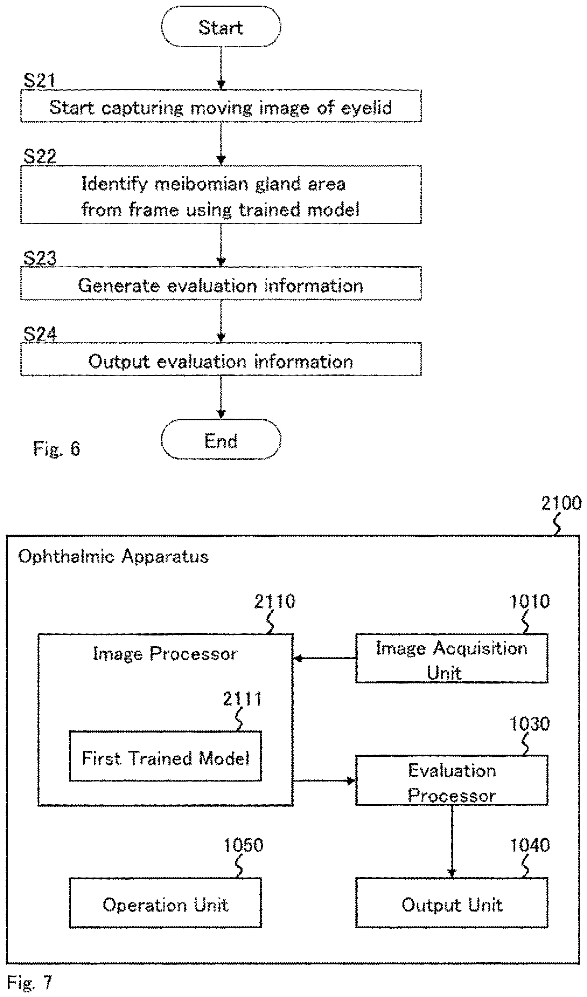

The operation of the ophthalmic apparatus 2000 according to the present embodiment example will now be described. An example of the operation is shown in FIG. 6. To begin with, the ophthalmic apparatus 2000 starts moving image photography of the subject's eyelid by the use of the image acquisition unit 1010 in the same manner as in the step S1 of FIG. 2 (S21).

Next, the ophthalmic apparatus 2000 identifies, by using the image processor 1021, a meibomian gland area from each of one or more frames of the moving image generated by the moving image photography started in the step S21 (S22).

At least a part of the processing of identifying a meibomian gland area in the present embodiment example is performed by using the trained model 1022. Several examples of the meibomian gland area identification performed by means of the trained model 1022 will be described later (refer to the second to sixth embodiment examples).

Next, the ophthalmic apparatus 2000 uses the evaluation processor 1030 to generate evaluation information relating to a predetermined disease based on the meibomian gland area identified in the step S22 in the same manner as in the step S3 of FIG. 2 (S23).

In the present operation example, the identification of the meibomian gland area (S22) and the generation of the evaluation information (S23) may be repeatedly performed, as in the operation example of FIG. 3. This repetition allows the evaluation information to be generated sequentially and in real time in parallel with the acquisition of the observation image (i.e., in parallel with the moving image photography). The plurality of pieces of evaluation information generated in this manner is stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2000 uses the output unit 1040 to output the evaluation information generated in the step S23 (S24). In the present step, at least one frame acquired by the moving image photography started in the step S21, information relating to the meibomian gland area identified in the step S22, or predetermined information other than these types of information may be output together with the evaluation information generated in the step S23. This completes the operation example shown in FIG. 6 (End).

According to the present embodiment example which is configured to be capable of performing the operation example of FIG. 6, a meibomian gland area can be identified from a frame of a moving image of the subject's eyelid, and then evaluation information can be generated. Therefore, compared to existing or conventional examination techniques or technologies by means of meibography, it is possible for the present embodiment example to provide an examination result relating to a meibomian gland more quickly.

In addition, according to the present embodiment example which is configured to be capable of performing the operation example of FIG. 6, a meibomian gland area can be identified using a trained model constructed by means of machine learning. Therefore, compared to existing or conventional examination techniques or technologies by means of meibography, it is possible for the present embodiment example to perform an examination relating to meibomian glands with higher quality (e.g., with higher accuracy, higher precision, etc.).

### < Second embodiment example>

A second embodiment example will now be described. The ophthalmic apparatus 2100 shown in FIG. 7 includes the image acquisition unit 1010 and the evaluation processor 1030, which are the same as or similarly configured to those of the ophthalmic apparatus 1000 shown in FIG. 1. The image processor 2110 according to the present embodiment example includes the first trained model 2111.

The first trained model 2111 does not need to be arranged inside the image processor 2110 and may be stored in a storage device that is directly or indirectly accessible by the image processor 2110.

The first trained model 2111 is an example of the trained model 1022 of the first embodiment example. The first trained model 2111 may be a part of the trained model 1022 of the first embodiment example.

The ophthalmic apparatus 2100 may include, as optional elements, the output unit 1040 and the operation unit 1050, which are the same as or similarly configured to those of the ophthalmic apparatus 1000 shown in FIG. 1. Although not shown in the drawings, the ophthalmic apparatus 2100 may include various kinds of processors, various kinds of storage devices, and other elements.

The image processor 2110 is configured to perform, by using the first trained model 2111, a part of the processing of identifying a meibomian gland area from a frame of a moving image of the subject's eyelid acquired by the image acquisition unit 1010.

More specifically, the image processor 2110 is configured to perform, by using the first trained model 2111, the processing of determining an analysis target area from a frame of a moving image of the subject's eyelid acquired by the image acquisition unit 1010. Furthermore, the image processor 2110 is configured to perform the processing of identifying a meibomian gland area from the analysis target area based on the analysis target area identified by using the first trained model 2111.

With regard to the processing of identifying a meibomian gland area from the analysis target area, the entirety of the processing may be performed without using a trained model, or a part or the entirety of the processing may be performed by using a trained model.

In the case where the processing of identifying a meibomian gland area from the analysis target area is performed without using a trained model, a meibomian gland area may be extracted from the analysis target area by employing the aforementioned techniques and technologies disclosed in Japanese Unexamined Patent Application Publication No. 2012-217621 (Patent Document 2), for example.

Several examples of the cases where a part or the entirety of the processing of identifying a meibomian gland area from the analysis target area is performed by using a trained model will be described later (refer to the fourth and fifth embodiment examples).

The first trained model 2111 according to the present embodiment example will now be described. The first trained model 2111 is constructed by means of machine learning using training data that includes eyelid images. The training data in the present embodiment example includes, for example, a label assigned to each eyelid image by annotation. The label includes information indicating the analysis target area in the eyelid image, for example.

The first trained model 2111 constructed by using such training data is a mathematical model that has been constructed by using machine learning so as to receive an input of a frame (an eyelid image) of a moving image of the subject's eyelid acquired by the image acquisition unit 1010 and to output an analysis target area, which is information indicating the range of the analysis target area, in this frame.

The image processor 2110 of the present embodiment example identifies a meibomian gland area in this frame based on the analysis target area determined by using the first trained model 2111.

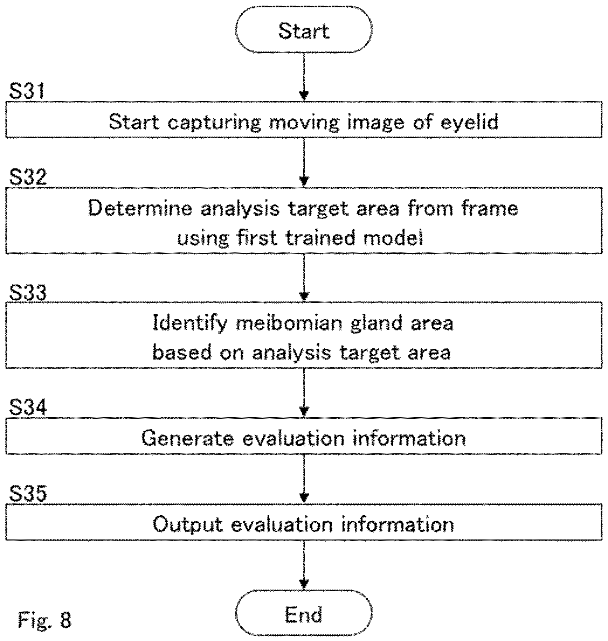

The operation of the ophthalmic apparatus 2100 according to the present embodiment example will now be described. An example of the operation is shown in FIG. 8. To begin with, the ophthalmic apparatus 2100 starts moving image photography of the subject's eyelid by the use of the image acquisition unit 1010 in the same manner as in the step S1 of FIG. 2 (S31).

Next, the ophthalmic apparatus 2100 determines an analysis target area from each of one or more frames of the moving image generated by the moving image photography started in the step S31 (S32). This determination processing is performed by the image processor 2110 using the first trained model 2111.

Next, based on the analysis target area determined in the step S32, the ophthalmic apparatus 2100 identifies, by using the image processor 2110, a meibomian gland area in this analysis target area (S33).

Next, based on the meibomian gland area identified in the step S33, the ophthalmic apparatus 2100 generates, by using the evaluation processor 1030, evaluation information relating to a predetermined disease in the same manner as in the step S3 of FIG. 2 (S34).

**In** the present operation example, the determination of the analysis target area (S32), the identification of the meibomian gland area (S33), and the generation of the evaluation information (S34) may be repeatedly performed, as in the operation example of FIG. 3. This repetition allows the evaluation information to be generated sequentially and in real time in parallel with the acquisition of the observation image (i.e., in parallel with the moving image photography). The plurality of pieces of evaluation information generated in this manner are stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2100 uses the output unit 1040 to output the evaluation information generated in the step S34 (S35). **In** the present step, at least one frame acquired by the moving image photography started in the step S31, information relating to the analysis target area determined in the step S32, information relating to the meibomian gland area identified in the step S33, or predetermined information other than these types of information may be output together with the evaluation information generated in the step S34. This completes the operation example shown in FIG. 8 (End).

According to the present embodiment example which is configured to be capable of performing the operation example of FIG. 8, a meibomian gland area can be identified from a frame of a moving image of the subject's eyelid and then evaluation information can be generated. Therefore, compared to existing or conventional examination techniques or technologies by means of meibography, it is possible for the present embodiment example to provide an examination result relating to a meibomian gland more quickly.

Furthermore, according to the present embodiment example which is configured to be capable of performing the operation example of FIG. 8, an analysis target area can be determined by using a trained model constructed through machine learning, and then a meibomian gland area can be identified based on the analysis target area. Therefore, compared to existing or conventional examination techniques or technologies by means of meibography, it is possible for the present embodiment example to perform an examination relating to meibomian glands with higher quality (e.g., with higher accuracy, higher precision, etc.).

### < Third embodiment example >

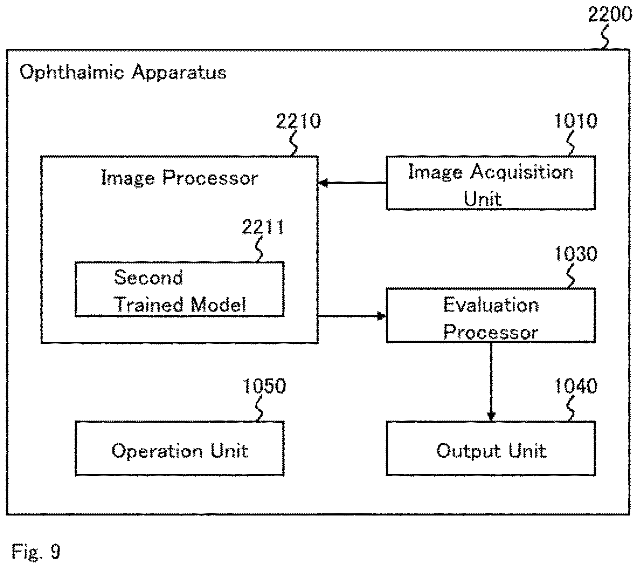

The third embodiment example will now be described. The ophthalmic apparatus 2200 shown in FIG. 9 includes the image acquisition unit 1010 and the evaluation processor 1030, which are the same as or similarly configured to those of the ophthalmic apparatus 1000 shown in FIG. 1. The image processor 2210 according to the present embodiment example includes the second trained model 2211.

It should be noted that the second trained model 2211 does not need to be arranged inside the image processor 2210 and may be stored in a storage device that is directly or indirectly accessible by the image processor 2210.

The second trained model 2211 is an example of the trained model 1022 of the first embodiment example. The second trained model 2211 may be a part of the trained model 1022 of the first embodiment example.

The ophthalmic apparatus 2200 may include, as optional elements, the output unit 1040 and the operation unit 1050, which are the same as or similarly configured to those of the ophthalmic apparatus 1000 shown in FIG. 1. Although not shown in the drawings, the ophthalmic apparatus 2200 may include various kinds of processors, various kinds of storage devices, and other elements.

The image processor 2210 performs, by using the second trained model 2211, a part of the processing of identifying a meibomian gland area from a frame of a moving image of the subject's eyelid acquired by the image acquisition unit 1010.

More specifically, the image processor 2210 is configured to perform, by using the second trained model 2211, segmentation of dividing a frame of a moving image of the subject's eyelid acquired by the image acquisition unit 1010 into a plurality of image areas (a plurality of segments). Furthermore, the image processor 2210 is configured to identify meibomian gland areas from the frame, based on the plurality of image areas obtained by the segmentation performed using the second trained model 2211.

With regard to the processing of identifying a meibomian gland area from the plurality of image areas, the entirety of the processing may be performed without using a trained model, or a part or the entirety of the processing may be performed by using a trained model.

**In** the case where the processing of identifying meibomian gland areas from the plurality of image areas is performed without using a trained model, for example, the aforementioned techniques and technologies disclosed in Japanese Unexamined Patent Application Publication No. 2012-217621 (Patent Document 2) may be used to identify meibomian gland areas from the plurality of image areas obtained by the segmentation executed using the second trained model 2211.

**In** the case where the processing of identifying meibomian gland areas from the plurality of image areas is performed by using a trained model, the trained model may be, for example, a mathematical model that has been trained to determine whether or not an image area is a meibomian gland area on the basis of the aspect (e.g., shape, size, position, relative position, or the like) of the image area.

The machine learning executed for constructing this trained model is performed by using training data that includes, for example, a training image including at least one image area and also includes a label assigned to the image area in the training image. Here, the label assigned to the image area in the training image may be a label indicating that the image area is a meibomian gland area, or a label indicating that the image area is not a meibomian gland area.

The second trained model 2211 according to the present embodiment example will now be described. The second trained model 2211 may be a mathematical model for performing a known segmentation method. The segmentation method may be, for example, any one of semantic segmentation, panoptic segmentation, and instance segmentation, or a combination of any two or more of these. Alternatively, the segmentation method may be a method configured to utilize another segmentation method at least in part. The training data used in the machine learning to construct the second trained model 2211 may be training data created according to the segmentation method to be employed.

The second trained model 2211 constructed in this manner is a mathematical model that has been constructed by using machine learning so as to receive an input of a frame (an eyelid image) of a moving image of the subject's eyelid acquired by the image acquisition unit 1010 and to output a plurality of image areas, each of which is information indicating the range of each image area, in this frame.

The image processor 2210 of the present embodiment example identifies a meibomian gland area in this frame based on the plurality of image areas determined using the second trained model 2211.

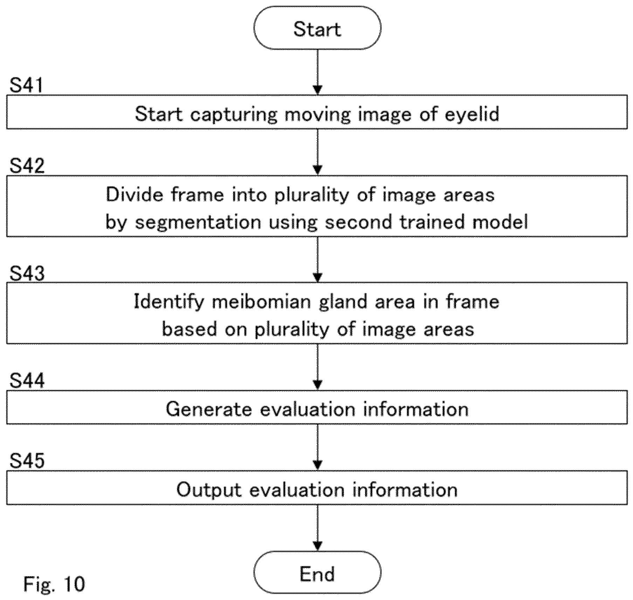

The operation of the ophthalmic apparatus 2200 according to the present embodiment example will now be described. An example of the operation is shown in FIG. 10. To begin with, the ophthalmic apparatus 2200 starts moving image photography of the subject's eyelid by the use of the image acquisition unit 1010 in the same manner as in the step S1 of FIG. 2 (S41).

Next, the ophthalmic apparatus 2200 applies segmentation to each of one or more frames of the moving image generated by the moving image photography started in the step S41, to divide the frame into a plurality of image areas (a plurality of segments) (S42). This segmentation is performed by the image processor 2210 using the second trained model 2211.

Next, the ophthalmic apparatus 2200 identifies, by using the image processor 2210, a meibomian gland area in the frame based on the plurality of image areas obtained in the step S42 (S43).

Next, the ophthalmic apparatus 2200 uses the evaluation processor 1030 to generate evaluation information relating to a predetermined disease based on the meibomian gland area identified in the step S43 in the same manner as in the step S3 of FIG. 2 (S44).

In the present operation example, the segmentation (S42), the identification of the meibomian gland area (S43), and the generation of the evaluation information (S44) may be repeatedly performed, as in the operation example of FIG. 3. This repetition allows the evaluation information to be generated sequentially and in real time in parallel with the acquisition of the observation image (i.e., in parallel with the moving image photography). The plurality of pieces of evaluation information generated in this manner are stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2200 uses the output unit 1040 to output the evaluation information generated in the step S44 (S45). In the present step, at least one frame acquired by the moving image photography started in the step S41, information relating to the plurality of image areas obtained in the step S42, information relating to the meibomian gland area identified in the step S43, or predetermined information other than these types of information may be output together with the evaluation information generated in the step S44. This completes the operation example shown in FIG. 10 (End).

According to the present embodiment example which is configured to be capable of performing the operation example of FIG. 10, a meibomian gland area can be identified from a frame of a moving image of the subject's eyelid and then evaluation information can be generated. Therefore, compared to existing or conventional examination techniques or technologies by means of meibography, it is possible for the present embodiment example to provide an examination result relating to a meibomian gland more quickly.

Furthermore, according to the present embodiment example which is configured to be capable of performing the operation example of FIG. 10, it is possible to divide a frame into a plurality of image areas by means of segmentation performed using a trained model constructed by means of machine learning, and to identify a meibomian gland area based on the plurality of image areas obtained. Therefore, compared to existing or conventional examination techniques or technologies by means of meibography, it is possible for the present embodiment example to perform an examination relating to meibomian glands with higher quality (e.g., with higher accuracy, higher precision, etc.).

### < Fourth embodiment example >

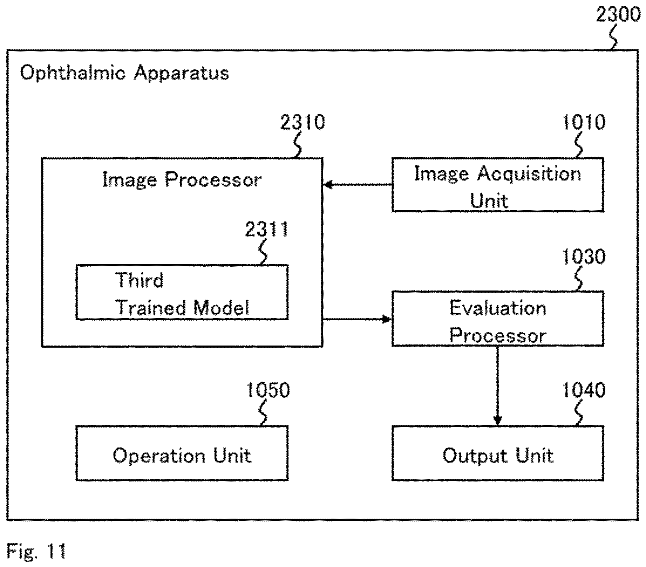

The fourth embodiment example will now be described. The ophthalmic apparatus 2300 shown in FIG. 11 includes the image acquisition unit 1010 and the evaluation processor 1030, which are the same as or similarly configured to those of the ophthalmic apparatus 1000 shown in FIG. 1. The image processor 2310 according to the present embodiment example includes the third trained model 2311.

It should be noted that the third trained model 2311 does not need to be arranged inside the image processor 2310 and may be stored in a storage device that is directly or indirectly accessible by the image processor 2310.

The third trained model 2311 is an example of the trained model 1022 of the first embodiment example. The third trained model 2311 may be a part of the trained model 1022 of the first embodiment example.

The ophthalmic apparatus 2300 may include, as optional elements, the output unit 1040 and the operation unit 1050, which are the same as or similarly configured to those of the ophthalmic apparatus 1000 shown in FIG. 1. Although not shown in the drawings, the ophthalmic apparatus 2300 may include various kinds of processors, various kinds of storage devices, and other elements.

The image processor 2310 performs, by using the third trained model 2311, a part of the processing of identifying a meibomian gland area from a frame of a moving image of the subject's eyelid acquired by the image acquisition unit 1010.

More specifically, the image processor 2310 is configured to perform the processing of determining an analysis target area from a frame of a moving image of the subject's eyelid acquired by the image acquisition unit 1010. Furthermore, the image processor 2310 is configured to perform segmentation of dividing the analysis target area into a plurality of image areas (a plurality of segments) by using the third trained model 2311. **In** addition, the image processor 2310 is configured to identify a meibomian gland area from the analysis target area based on the plurality of image areas obtained by the segmentation performed using the third trained model 2311.

With regard to the processing of determining an analysis target area from a frame, the entirety of the processing may be performed without using a trained model, or a part or the entirety of the processing may be performed by using a trained model.

**In** the case where the processing of determining an analysis target area from a frame is performed without using a trained model, for example, the aforementioned techniques and technologies disclosed in Japanese Unexamined Patent Application Publication No. 2012-217621 (Patent Document 2) may be employed.

In the case where the processing of determining an analysis target area from a frame is performed by using a trained model, for example, the configuration according to the second embodiment example can be employed.

Similarly, with regard to the processing of identifying a meibomian gland area from the plurality of image areas, the entirety of the processing may be performed without using a trained model, or a part or the entirety of the processing may be performed by using a trained model.

In the case where the processing of identifying a meibomian gland area from the plurality of image areas is performed without using a trained model, for example, the aforementioned techniques and technologies disclosed in Japanese Unexamined Patent Application Publication No. 2012-217621 (Patent Document 2) may be used to identify a meibomian gland area from the plurality of image areas obtained by the segmentation executed using the third trained model 2311.

In the case where the processing of identifying a meibomian gland area from the plurality of image areas is performed by using a trained model, the trained model may be, for example, a mathematical model that has been trained to determine whether or not an image area is a meibomian gland area on the basis of the aspect (e.g., shape, size, position, relative position, or the like) of the image area, as in the similar case described in the third embodiment example. The machine learning for constructing such trained model is performed using training data that includes, for example, a training image including at least one image area and also includes a label assigned to the image area in the training image. Here, the label assigned to the image area in the training image is a label indicating that the image area is a meibomian gland area, or a label indicating that the image area is not a meibomian gland area.

The third trained model 2311 according to the present embodiment example will now be described. The third trained model 2311 may be a mathematical model for performing a known segmentation method. The segmentation method may be, for example, any one of semantic segmentation, panoptic segmentation, and instance segmentation, or a combination of any two or more of these. Alternatively, the segmentation method may be a method configured to utilize another segmentation method at least in part. The training data used in the machine learning to construct the third trained model 2311 may be training data created according to the segmentation method to be employed.

The third trained model 2311 constructed in this manner is a mathematical model that has been constructed by using machine learning so as to receive an input of an analysis target area in a frame (an eyelid image) of a moving image of the subject's eyelid acquired by the image acquisition unit 1010 and to output a plurality of image areas, each of which is information indicating the range of each image area, in this analysis target area.

The image processor 2310 of the present embodiment example identifies a meibomian gland area in this analysis target area based on the plurality of image areas determined using the third trained model 2311.

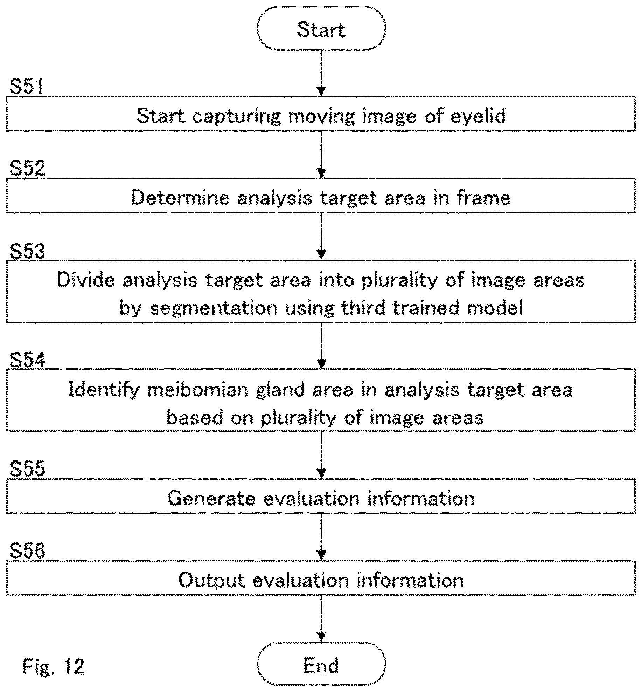

The operation of the ophthalmic apparatus 2300 according to the present embodiment example will now be described. An example of the operation is shown in FIG. 12. To begin with, the ophthalmic apparatus 2300 starts moving image photography of the subject's eyelid by the use of the image acquisition unit 1010 in the same manner as in the step S1 of FIG. 2 (S51).

Next, the ophthalmic apparatus 2300 uses the image processor 2310 to determine an analysis target area in each of one or more frames of the moving image generated by the moving image photography started in the step S51 (S52).

Next, the ophthalmic apparatus 2300 applies segmentation to each analysis target area determined in the step S52 to divide this analysis target area into a plurality of image areas (a plurality of segments) (S53). This segmentation is performed by the image processor 2310 using the third trained model 2311.

Next, the ophthalmic apparatus 2300 identifies, by using the image processor 2310, a meibomian gland area in each analysis target area based on the plurality of image areas obtained for this analysis target area in the step S53 (S54).

Next, the ophthalmic apparatus 2300 uses the evaluation processor 1030 to generate evaluation information relating to a predetermined disease based on the meibomian gland area identified in the step S54 in the same manner as in the step S3 of FIG. 2 (S55).

In the present operation example, the determination of the analysis target area (S52), the segmentation (S53), the identification of the meibomian gland area (S54), and the generation of the evaluation information (S55) may be repeatedly performed, as in the operation example of FIG. 3. This repetition makes it possible to generate evaluation information sequentially and in real time in parallel with the acquisition of the observation image (in parallel with the moving image photography). The plurality of pieces of evaluation information generated in this manner are stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2300 uses the output unit 1040 to output the evaluation information generated in the step S55 (S56). **In** the present step, at least one frame acquired by the moving image photography started in the step S51, information relating to the analysis target area determined in the step S52, information relating to the plurality of image areas obtained in the step S53, information relating to the meibomian gland area identified in the step S54, or predetermined information other than these types of information may be output together with the evaluation information generated in the step S55. This completes the operation example shown in FIG. 12 (End).

According to the present embodiment example which is configured to be capable of performing the operation example of FIG. 12, a meibomian gland area can be identified from a frame of a moving image of the subject's eyelid and then evaluation information can be generated. Therefore, compared to existing or conventional examination techniques or technologies by means of meibography, it is possible for the present embodiment example to provide an examination result relating to a meibomian gland more quickly.

Furthermore, according to the present embodiment example which is configured to be capable of performing the operation example of FIG. 12, it is possible to divide an analysis target area in a frame into a plurality of image areas by means of segmentation performed using a trained model constructed by means of machine learning, and to identify a meibomian gland area based on the plurality of image areas obtained. Therefore, compared to existing or conventional examination techniques or technologies by means of meibography, it is possible for the present embodiment example to perform an examination relating to meibomian glands with higher quality (e.g., with higher accuracy, higher precision, etc.).

### < Fifth embodiment example >

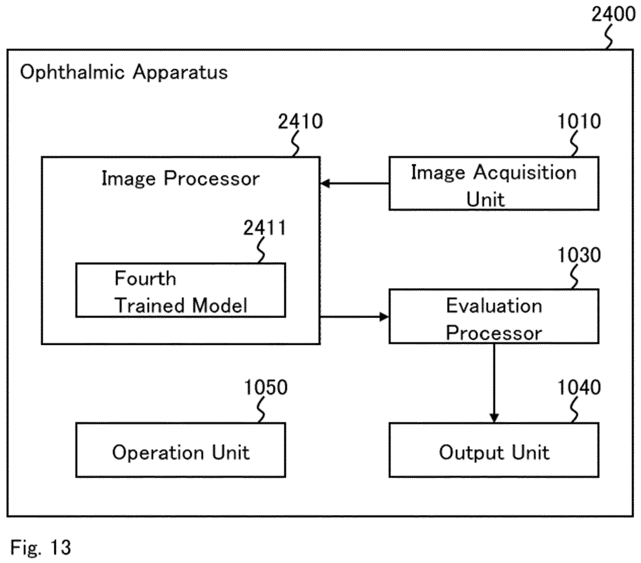

The fifth embodiment example will now be described. The ophthalmic apparatus 2400 shown in FIG. 13 includes the image acquisition unit 1010 and the evaluation processor 1030, which are the same as or similarly configured to those of the ophthalmic apparatus 1000 shown in FIG. 1. The image processor 2410 according to the present embodiment example includes the fourth trained model 2411.

It should be noted that the fourth trained model 2411 does not need to be arranged inside the image processor 2410 and may be stored in a storage device that is directly or indirectly accessible by the image processor 2410.

The fourth trained model 2411 is an example of the trained model 1022 of the first embodiment example. The fourth trained model 2411 may be a part of the trained model 1022 of the first embodiment example.

The ophthalmic apparatus 2400 may include, as optional elements, the output unit 1040 and the operation unit 1050, which are the same as or similarly configured to those of the ophthalmic apparatus 1000 shown in FIG. 1. Although not shown in the drawings, the ophthalmic apparatus 2400 may include various kinds of processors, various kinds of storage devices, and other elements.

The image processor 2410 performs, by using the fourth trained model 2411, a part of the processing of identifying a meibomian gland area from a frame of a moving image of the subject's eyelid acquired by the image acquisition unit 1010.

More specifically, the image processor 2410 is configured to perform the processing of determining an analysis target area from a frame of a moving image of the subject's eyelid acquired by the image acquisition unit 1010. Furthermore, the image processor 2410 is configured to perform the processing of identifying a meibomian gland area from the analysis target area by using the fourth trained model 2411.

With regard to the processing of determining an analysis target area from a frame, the entirety of the processing may be performed without using a trained model, or a part or the entirety of the processing may be performed by using a trained model.

**In** the case where the processing of determining an analysis target area from a frame is performed without using a trained model, for example, the aforementioned techniques and technologies disclosed in Japanese Unexamined Patent Application Publication No. 2012-217621 (Patent Document 2) may be employed.

**In** the case where the processing of determining an analysis target area from a frame is performed by using a trained model, for example, the configuration according to the second embodiment example can be employed.

The fourth trained model 2411 according to the present embodiment example will now be described. The fourth trained model 2411 may be a mathematical model for performing a known segmentation method. The segmentation method may be, for example, any one of semantic segmentation, panoptic segmentation, and instance segmentation, or a combination of any two or more of these. Alternatively, the segmentation method may be a method configured to utilize another segmentation method at least in part. The training data used in the machine learning to construct the fourth trained model 2411 may be training data created according to the segmentation method to be employed.

The fourth trained model 2411 constructed in this manner is a mathematical model that has been constructed by using machine learning so as to receive an input of an analysis target area in a frame (an eyelid image) of the moving image of the subject's eyelid acquired by the image acquisition unit 1010 and to output a meibomian gland area, which is information indicating the range of this meibomian gland area, in this analysis target area.

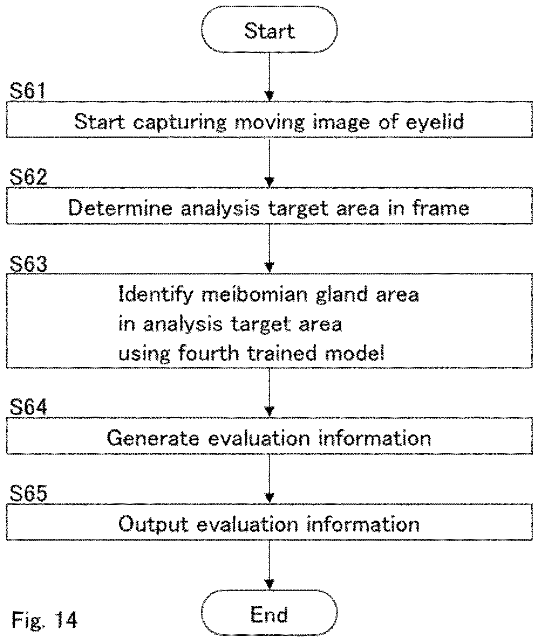

The operation of the ophthalmic apparatus 2400 according to the present embodiment example will now be described. An example of the operation is shown in FIG. 14. To begin with, the ophthalmic apparatus 2400 starts moving image photography of the subject's eyelid by the use of the image acquisition unit 1010 in the same manner as in the step S1 of FIG. 2 (S61).

Next, the ophthalmic apparatus 2400 uses the image processor 2410 to determine an analysis target area in each of one or more frames of the moving image generated by the moving image photography started in the step S61 (S62).

Next, the ophthalmic apparatus 2400 identifies a meibomian gland area in the analysis target area determined in the step S62 (S63). This identification is performed by the image processor 2410 using the fourth trained model 2411.

Next, in the same manner as in the step S3 of FIG. 2, the ophthalmic apparatus 2400 uses the evaluation processor 1030 to generate evaluation information relating to a predetermined disease based on the meibomian gland area identified in the step S63 (S64).

**In** the present operation example, the determination of the analysis target area (S62), the identification of the meibomian gland area (S63), and the generation of the evaluation information (S64) may be repeatedly performed, as in the operation example of FIG. 3. This repetition makes it possible to generate evaluation information sequentially and in real time in parallel with the acquisition of the observation image (in parallel with the moving image photography). The plurality of pieces of evaluation information generated in this manner are stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2400 uses the output unit 1040 to output the evaluation information generated in the step S64 (S65). In the present step, at least one frame acquired by the moving image photography started in the step S61, information relating to the analysis target area determined in the step S62, information relating to the meibomian gland area identified in the step S63, or predetermined information other than these types of information may be output together with the evaluation information generated in the step S64. This completes the operation example shown in FIG. 14 (End).

According to the present embodiment example which is configured to be capable of performing the operation example of FIG. 14, a meibomian gland area can be identified from a frame of a moving image of the subject's eyelid and then evaluation information can be generated. Therefore, compared to existing or conventional examination techniques or technologies by means of meibography, it is possible for the present embodiment example to provide an examination result relating to a meibomian gland more quickly.

Furthermore, according to the present embodiment example which is configured to be capable of performing the operation example of FIG. 14, a meibomian gland area can be identified from an analysis target area in a frame using a trained model constructed by means of machine learning. Therefore, compared to existing or conventional examination techniques or technologies by means of meibography, it is possible for the present embodiment example to perform an examination relating to meibomian glands with higher quality (e.g., with higher accuracy, higher precision, etc.).

### < Sixth embodiment example >

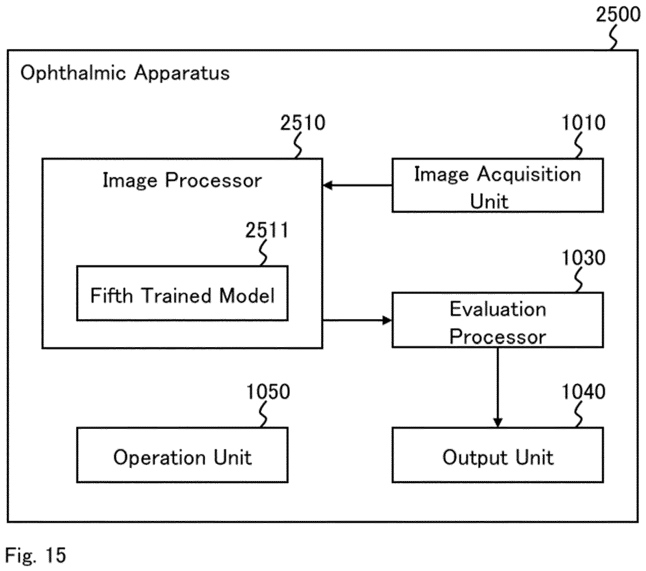

The sixth embodiment example will now be described. The ophthalmic apparatus 2500 shown in FIG. 15 includes the image acquisition unit 1010 and the evaluation processor 1030, which are the same as or similarly configured to those of the ophthalmic apparatus 1000 shown in FIG. 1. The image processor 2510 according to the present embodiment example includes the fifth trained model 2511.

It should be noted that the fifth trained model 2511 does not need to be arranged inside the image processor 2510 and may be stored in a storage device that is directly or indirectly accessible by the image processor 2510.

The fifth trained model 2511 is an example of the trained model 1022 of the first embodiment example. The fifth trained model 2511 may be a part of the trained model 1022 of the first embodiment example.

The ophthalmic apparatus 2500 may include, as optional elements, the output unit 1040 and the operation unit 1050, which are the same as or similarly configured to those of the ophthalmic apparatus 1000 shown in FIG. 1. Although not shown in the drawings, the ophthalmic apparatus 2500 may include various kinds of processors, various kinds of storage devices, and other elements.

The image processor 2410 performs, by using the fifth trained model 2511, the entirety of the processing of identifying a meibomian gland area from a frame of a moving image of the subject's eyelid acquired by the image acquisition unit 1010. In contrast, in the second to fourth embodiment examples, performed using a trained model is only a part of the processing of identifying a meibomian gland area from a frame of a moving image of the subject's eyelid acquired by the image acquisition unit 1010.

The fifth trained model 2511 according to the present embodiment example will now be described. The fifth trained model 2511 may be a mathematical model for performing a known segmentation method. The segmentation method may be, for example, any one of semantic segmentation, panoptic segmentation, and instance segmentation, or a combination of any two or more of these. Alternatively, the segmentation method may be a method configured to utilize another segmentation method at least in part. The training data used in the machine learning to construct the fifth trained model 2511 may be training data created according to the segmentation method to be employed.

The fifth trained model 2511 may include two or more mathematical models (two or more trained models). Several examples of the fifth trained model 2511 thus configured are described below.

In one example, the fifth trained model 2511 may include the following trained models: a trained model that has been trained to determine an analysis target area from a frame of a moving image of the subject's eyelid acquired by the image acquisition unit 1010; and a trained model that has been trained to identify a meibomian gland area by applying segmentation to the analysis target area determined.

**In** another example, the fifth trained model 2511 may include the following trained models: a trained model that has been trained to determine an analysis target area from a frame of a moving image of the subject's eyelid acquired by the image acquisition unit 1010; a trained model that has been trained to divide the analysis target area determined into a plurality of image areas by applying segmentation to the analysis target area determined; and a trained model that has been trained to identify a meibomian gland area based on the plurality of image areas obtained.

**In** yet another example, the fifth trained model 2511 may include the following trained models: a trained model that has been trained to apply segmentation to a frame of a moving image of the subject's eyelid acquired by the image acquisition unit 1010 to divide the frame into a plurality of image areas; and a trained model that has been trained to identify a meibomian gland area based on the plurality of image areas obtained.

The fifth trained model 2511 constructed in the above manner is a mathematical model that has been constructed by using machine learning so as to receive an input of a frame (an eyelid image) of the moving image of the subject's eyelid acquired by the image acquisition unit 1010 and to output a meibomian gland area, which is information indicating the range of the meibomian gland area, in the entirety of the frame.

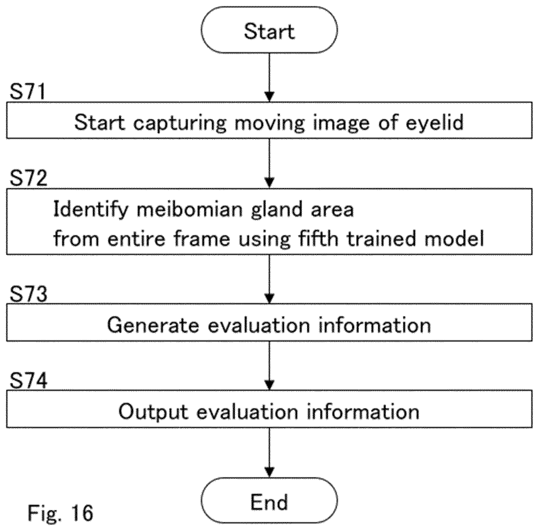

The operation of the ophthalmic apparatus 2500 according to the present embodiment example will now be described. An example of the operation is shown in FIG. 16. To begin with, the ophthalmic apparatus 2500 starts moving image photography of the subject's eyelid by the use of the image acquisition unit 1010 in the same manner as in the step S1 of FIG. 2 (S71).

Next, the ophthalmic apparatus 2500 identifies a meibomian gland area from the entirety of one or more frames of the moving image generated by the moving image photography started in the step S71 (S72). This identification is performed by the image processor 2510 using the fifth trained model 2511.

Next, in the same manner as in the step S3 of FIG. 2, the ophthalmic apparatus 2500 uses the evaluation processor 1030 to generate evaluation information relating to a predetermined disease based on the meibomian gland area identified in the step S72 (S73).

In the present operation example, the identification of the meibomian gland area (S72) and the generation of the evaluation information (S73) may be repeatedly performed, as in the operation example of FIG. 3. This repetition makes it possible to generate evaluation information sequentially and in real time in parallel with the acquisition of the observation image (in parallel with the moving image photography). The plurality of pieces of evaluation information generated in this manner are stored in a storage device which is not shown in the drawings. The utilization method of the plurality of pieces of evaluation information may be freely selected or determined, and it may be, for example, the utilization method described in the operation example of FIG. 3.

Finally, the ophthalmic apparatus 2500 uses the output unit 1040 to output the evaluation information generated in the step S73 (S74). In the present step, at least one frame acquired by the moving image photography started in the step S71, information relating to the meibomian gland area identified in the step S72, or predetermined information other than these types of information may be output together with the evaluation information generated in the step S73. This completes the operation example shown in FIG. 16 (End).

According to the present embodiment example which is configured to be capable of performing the operation example of FIG. 16, a meibomian gland area can be identified from a frame of a moving image of the subject's eyelid and then evaluation information can be generated. Therefore, compared to existing or conventional examination techniques or technologies by means of meibography, it is possible for the present embodiment example to provide an examination result relating to a meibomian gland more quickly.

Furthermore, according to the present embodiment example which is configured to be capable of performing the operation example of FIG. 16, a meibomian gland area can be identified from the entirety of the frame using a trained model constructed by means of machine learning. Therefore, compared to existing or conventional examination techniques or technologies by means of meibography, it is possible for the present embodiment example to perform an examination relating to meibomian glands with higher quality (e.g., with higher accuracy, higher precision, etc.).

### < Effects >

Some advantageous effects of an ophthalmic apparatus (1000, 2000, 2100, 2200, 2300, 2400, 2500) according to the exemplary embodiments will now be described. It should be noted that advantageous effects of an ophthalmic apparatus according to the embodiments are not limited to the matters and items described in the present disclosure.

The ophthalmic apparatus (1000, 2000, 2100, 2200, 2300, 2400, 2500) according to the embodiments includes a moving image acquisition unit (the image acquisition unit 1010), an image processor (the image processors 1020, 1021, 2110, 2210, 2310, 2410, 2510), and an evaluation processor (the evaluation processor 1030).

The moving image acquisition unit is configured to acquire a moving image of an eyelid of the subject.

The image processor is configured to perform processing of identifying a meibomian gland area from at least one frame of the moving image acquired by the moving image acquisition unit.

The evaluation processor is configured to perform processing of generating evaluation information relating to a predetermined disease based on the meibomian gland area identified by the image processor.

The ophthalmic apparatus according to the embodiments configured in this manner is capable of identifying a meibomian gland area from a frame of the moving image of the subject's eyelid and then generating evaluation information. In contrast, in existing or conventional examinations conducted using meibography, it is necessary to go through the following steps: a step of photographing the back surface of the eyelid; a step of storing the still image obtained; and a step of analyzing and evaluating the still image. In comparison to such existing or conventional techniques or technologies requiring a series of steps described above, the ophthalmic apparatus according to the embodiments has an advantageous effect of being able to provide an examination result relating to a meibomian gland more quickly.

In the ophthalmic apparatus (1000, 2000, 2100, 2200, 2300, 2400, 2500) according to the embodiments, the image processor (1020, 1021, 2110, 2210, 2310, 2410, 2510) may be configured to sequentially processing a plurality of frames of the moving image of the subject's eyelid acquired by the moving image acquisition unit (the image acquisition unit 1010), thereby identifying a meibomian gland area from each of the plurality of frames.

It should be noted that the plurality of frames to which this meibomian gland area identification processing is applied may be all of the frames acquired by the moving image acquisition unit, or may be frames selected from all of the frames by, for example, thinning processing.

The ophthalmic apparatus according to the embodiments configured in this manner is capable of identifying a meibomian gland area in real time from a frame of a moving image of the subject's eyelid and generate evaluation information, making it possible to provide an examination result relating to a meibomian gland more quickly than with the existing or conventional techniques or technologies which require the series of steps described above.

In the ophthalmic apparatus (2000, 2100, 2200, 2300, 2400, 2500) according to the embodiments, the image processor (1021, 2110, 2210, 2310, 2410, 2510) may be configured to perform at least a part of the processing of identifying a meibomian gland area from a frame of the moving image of the subject's eyelid acquired by the moving image acquisition unit (the image acquisition unit 1010), by using a trained model (1022, 2111, 2211, 2311, 2411, 2511) that has been constructed by machine learning with training data including an eyelid image.

The ophthalmic apparatus according to the embodiments configured in this way has an advantageous effect of being able to provide an examination result relating to a meibomian gland more quickly than with the existing or conventional examination techniques or technologies conducted using meibography. In addition, since the ophthalmic apparatus according to the embodiments configured in this way is capable of identifying a meibomian gland area by using a trained model constructed by means of machine learning, it is possible to achieve a further advantageous effect of being able to perform an examination relating to meibomian glands with higher quality (e.g., with higher accuracy, higher precision, etc.) than with the existing or conventional examination techniques or technologies conducted using meibography.

The image processor (2110) according to some aspect examples is configured to perform processing of determining an analysis target area from a frame of the moving image of the subject's eyelid acquired by the moving image acquisition unit (the image acquisition unit 1010) by using the trained model (2111), and to further identify a meibomian gland area based on the analysis target area determined. The present aspect is an example of the cases in which only a part of the processing of identifying a meibomian gland area from a frame of the moving image of the subject's eyelid is performed using a trained model.

The image processor (2210, 2310) according to some aspect examples is configured to perform segmentation of dividing at least a part of a frame of the moving image of the subject's eyelid acquired by the moving image acquisition unit (the image acquisition unit 1010) into a plurality of image areas using the trained model (2211, 2311), and to further identify a meibomian gland area based on the plurality of image areas obtained. The present aspect is an example of the cases in which only a part of the processing of identifying a meibomian gland area from a frame of the moving image of the subject's eyelid is performed using a trained model.

The image processor (2410) according to some aspect examples is configured to use a trained model (2411) to perform processing of identifying a meibomian gland area from a part of a frame of the moving image of the subject's eyelid acquired by the moving image acquisition unit (the image acquisition unit 1010) using the trained mode (2411). The present aspect is an example of the cases in which only a part of the processing of identifying a meibomian gland area from a frame of the moving image of the subject's eyelid is performed using a trained model.

The image processor (2510) according to some aspect examples is configured to perform processing of identifying a meibomian gland area from the entirety of a frame of the moving image of the subject's eyelid acquired by the moving image acquisition unit (the image acquisition unit 1010) using the trained mode (2511). The present aspect is an example of the cases in which the entirety of the processing of identifying a meibomian gland area from a frame of the moving image of the subject's eyelid is performed using a trained model.

### < Method of processing ophthalmic image >

According to the ophthalmic apparatus of the various aspects described above, it is possible to implement a method of processing an ophthalmic image.

The method of processing an ophthalmic image is configured to perform processing of identifying a meibomian gland area from at least one frame of a moving image of a subject's eyelid, and to perform processing of generating evaluation information relating to a predetermined disease based on the meibomian gland area identified. Here, the step of identifying a meibomian gland area is performed by a first computer, and the step of generating evaluation information is performed by a second computer. The first computer and the second computer may be the same computer or different computers.

Any of the matters or items described in the present disclosure with respect to the ophthalmic apparatuses can be incorporated or combined with the method of processing an ophthalmic image of the present embodiment.

For example, in some aspect examples, in the step of identifying a meibomian gland area from a frame of the moving image of the subject's eyelid, a meibomian gland area can be identified from each of the plurality of frames of the moving image by sequentially processing the plurality of frames.

Further, in some aspect examples, at least a part of the processing of identifying a meibomian gland area from a frame of the moving image of the subject's eyelid can be performed by using a trained model constructed by means of machine learning with training data including an eyelid image.

Further, in some aspect examples, the processing of determining an analysis target area from a frame of the moving image of the subject's eyelid can be performed using a trained model, and a meibomian gland area can be identified based on the analysis target area determined.

Further, in some aspect examples, segmentation of dividing at least a part of a frame of the moving image of the subject's eyelid into a plurality of image areas can be performed using a trained model, and a meibomian gland area can be identified based on the plurality of image areas obtained.

Further, in some aspect examples, the processing of identifying a meibomian gland area from a part of a frame of the moving image of the subject's eyelid can be performed using a trained model.

Further, in some aspect examples, the processing of identifying a meibomian gland area from the entirety of a frame of the moving image of the subject's eyelid can be performed using a trained model.

According to the method of processing an ophthalmic image of the present embodiment configured as described above, it is possible to identify a meibomian gland area from a frame of a moving image of the subject's eyelid and generate evaluation information. **In** contrast, in existing or conventional examination methods using meibography, it is necessary to go through the following steps: a step of photographing the back surface of the eyelid; a step of storing the still image obtained; and a step of analyzing and evaluating the still image. **In** comparison to the existing or conventional techniques or technologies which require the series of steps described above, the method of processing an ophthalmic image according to the present embodiment has an advantageous effect of being able to provide an examination result relating to a meibomian gland more quickly.

In addition, the method of processing an ophthalmic image according to the present embodiment may be configured to perform meibomian gland area identification using a trained model constructed by means of machine learning. This method can achieve an advantageous effect of being able to perform an examination relating to meibomian glands with higher quality (e.g., with higher accuracy, higher precision, etc.) than with the existing or conventional examination techniques or technologies conducted using meibography.

By combining any of the matters or items described in the present disclosure regarding the ophthalmic apparatuses with the method of processing an ophthalmic image according to the present embodiment, it becomes possible for the present embodiment to provide an advantageous effect corresponding to the matters or items combined.

### < Program >

It is possible to configure a program that causes a computer to perform one or more pieces of processing freely selected or determined from the processing described in the present disclosure.

For example, the program according to some embodiments is a program that causes a computer to perform processing of an ophthalmic image and that is configured to cause the computer to perform the following pieces of processing: processing of identifying a meibomian gland area from at least one frame of a moving image of a subject's eyelid; and processing of generating evaluation information relating to a predetermined disease based on the meibomian gland area identified.

The program according to the present embodiment configured in this manner makes it possible to identify a meibomian gland area from a frame of the moving image of the subject's eyelid and generate evaluation information, making it possible to provide an examination result relating to a meibomian gland more quickly than with the existing or conventional examination techniques or technologies conducted using meibography.

Any of the matters or items described in the present disclosure regarding the ophthalmic apparatuses and/or the methods of processing an ophthalmic image can be combined with the program of the present embodiment. Such a program can achieve an advantageous effect corresponding to the mattes and items combined.

For example, by configuring the program to perform meibomian gland area identification using a trained model constructed by means of machine learning, it becomes possible to perform an examination relating to meibomian glands with higher quality (e.g., with higher accuracy, higher precision, etc.) than with the existing or conventional examination techniques or technologies conducted using meibography.

### < Recording medium >

A recording medium in which a program is recorded can be created, wherein the program is configured to cause a computer to perform one or more pieces of processing freely selected or determined from the processing described in the present disclosure. The recording medium is a non-transitory recording medium that can be read by a computer. Such a recording medium may be in any form. Examples of the recording medium include a magnetic disk, an optical disk, a magneto-optical disk, a semiconductor memory, and any other kinds of recording media.

The recording medium according to the present embodiment configured in this way makes it possible to identify a meibomian gland area from a frame of the moving image of the subject's eyelid and generate evaluation information, making it possible to provide an examination result relating to a meibomian gland more quickly than with the existing or conventional examination techniques or technologies conducted using meibography.

Any of the matters or items described in the present disclosure regarding any of the ophthalmic apparatuses, the methods of processing an ophthalmic image, and the programs can be combined with the recording medium of the present embodiment. Such a recording medium can achieve an advantageous effect corresponding to the matters or items combined.

For example, a recording medium in which a program configured to perform meibomian gland area identification using a trained model constructed by means of machine learning is recorded, makes it possible to perform an examination relating to meibomian glands with higher quality (e.g., with higher accuracy, higher precision, etc.) than with the existing or conventional examination techniques or technologies conducted using meibography.

The present disclosure presents several aspect examples of the embodiments. These aspects are merely examples of the present invention, and therefore any modifications (e.g., omissions, substitutions, additions, or the like) within the scope of the present invention can be applied to the present disclosure.

## Claims

1. An ophthalmic apparatus comprising:
a moving image acquisition unit configured to acquire a moving image of a subject's eyelid;
an image processor configured to perform processing of identifying a meibomian gland area from at least one frame of the moving image; and
an evaluation processor configured to perform processing of generating evaluation information relating to a predetermined disease based on the meibomian gland area.

2. The ophthalmic apparatus according to claim 1, wherein the image processor identifies a meibomian gland area from each of plurality of frames of the moving image by sequentially processing the plurality of frames.

3. The ophthalmic apparatus according to claim 1, wherein the image processor performs at least a part of the processing of identifying the meibomian gland area from a frame of the moving image by using a trained model constructed by means of machine learning with training data including an eyelid image.

4. The ophthalmic apparatus according to claim 3, wherein the image processor performs processing of determining an analysis target area from the frame using the trained model, and identifies a meibomian gland area based on the analysis target area.

5. The ophthalmic apparatus according to claim 3, wherein the image processor performs segmentation of dividing at least a part of the frame into a plurality of image areas using the trained model, and identifies a meibomian gland area based on the plurality of image areas.

6. The ophthalmic apparatus according to claim 3, wherein the image processor performs processing of identifying a meibomian gland area from a part of the frame using the trained model.

7. The ophthalmic apparatus according to claim 3, wherein the image processor performs processing of identifying a meibomian gland area from an entirety of the frame using the trained model.

8. A method of processing an ophthalmic image, the method comprising:
performing processing of identifying a meibomian gland area from at least one frame of a moving image of a subject's eyelid; and
performing processing of generating evaluation information relating to a predetermined disease based on the meibomian gland area.

9. A computer-readable non-transitory recording medium in which a program is recorded, the program being configured to cause a computer to perform processing of an ophthalmic image, wherein the program is configured to cause the computer to perform:
processing of identifying a meibomian gland area from at least one frame of a moving image of a subject's eyelid; and
processing of generating evaluation information relating to a predetermined disease based on the meibomian gland area.
